# EUROPEAN PATENT APPLICATION

(11) **EP 1 652 424 A1**
(43) Date of publication of application: **03.05.2006**
(21) Application number: 04747964.7
(22) Date of filing: 27.07.2004
(51) Int. Cl.: A01H 1/02, A01H 5/00

(54) **METHOD OF BREEDING AZALEA**

(30) Priority: 29.07.2003 JP 2003281527
(71) Applicant: Sakata, Yusuke, Kagoshima-shi, Kagoshima 890-0053 (JP); Hashimoto, Fumio, Kagoshima-shi, Kagoshima 890-0081 (JP)
(72) Inventor: Sakata, Yusuke, Kagoshima-shi, Kagoshima 890-0053 (JP); Hashimoto, Fumio, Kagoshima-shi, Kagoshima 890-0081 (JP)
(74) Representative: Strehl Schübel-Hopf & Partner
(86) International application number: PCT/JP2004/010651
(87) International publication number: WO 2005/009116

(57) **Abstract**

It is intended to provide a method of constructing an azalea plant whereby a gene relating to evergreen characteristics can be transferred into a deciduous azalea plant, a gene relating to heat tolerance can be transferred into a heat-intolerant azalea plant, or a gene relating to ever-blooming characteristics can be transferred into a one season blooming azalea plant. A gene relating to evergreen characteristics can be transferred into a deciduous azalea plant by crossing a deciduous azalea plant with an evergreen azalea plant. A gene relating to heat tolerance can be transferred into a heat-intolerant azalea plant by crossing a heat-intolerant azalea plant with a heat-intolerant azalea plant. A gene relating to ever-blooming characteristics can be transferred into a one season blooming azalea plant by crossing a one season blooming gene with an ever-blooming azalea plant.

## Description

### Technical Field

The present invention relates to a new method for breeding an azalea which introduce into an azalea that is deciduous, non-heat resistant, and one season flowering a gene concerning evergreen property, heat resistance, and ever flowering. More specifically, the invention relates to novel plants and a treatment for obtaining the same comprising flowers of glowering plants, i.e., angiosperms and a crossing method which is a treatment for altering genotype. The present invention also includes a method for partially utilizing a breeding process containing sexual hybridization. The present invention is also directed to new plants, which are flowering plants such as (angiosperms and a method for obtaining the same.

### Background Arts

Genus Rhododendron comprising five subtribes and approximately 850 types is distributed to from a low mountain to high mountain regions from the Torrid Zone to cold zone'. It makes up a characteristic group including enormous variations including deciduous type to evergreen type or creeping type to tall tree type from morphological and ecological viewpoints (Non-Patent Document 1: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991).

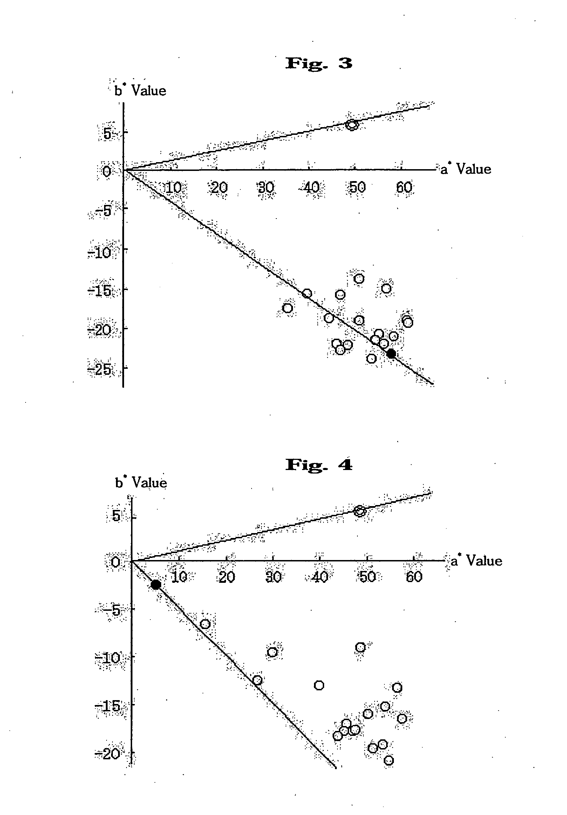

Amongst genus Rhododendron, what are highly evaluated and are differentiated into various varieties are four subtribes; evergreen azalea, deciduous azalea, non-scale rhododendron, and scale rhododendron. The reason why they are differentiated into various varieties is the fact that since their genomic differentiation within the genus is not so large, gene exchange between hetero geneities have been fairly, freely conducted and that even if the crossing is repeated, the resulting hybrids do not become sterility (Non-Patent Document 2: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991, FIG. 4).

Breeding of azalea is within the category of gene exchange between near species and does not deviate from the range of character alternation. Especially it is the present national and international situation that there is absolutely no breeding of novel azaleas involving alternation of ecotype (Non-Patent Document 1: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991)

From horticultural viewpoint, azalea has merits and demerits. For example, as for evergreen azaleas, they are relatively are easily cultured, but they lack yellow and true blue in their color variation. As for deciduous azalea, although strong yellow follower exists in exbury azalea, only in the case of R. dilatatum, which is typical Japanese azalea, the flower color is restricted to cinnabar red and purple red, and they have no heat resistance (Non Patent Document 3: Kennichi Arisumi, "Taishosei Shakunage no Ikushu (3)", Rhododendron 2003, Vol. 3, No. 1, p67-83).

In order to create new horticultural species widely utilizing enormous gene resource, breeding considering exotic germplasm, which has not been deeply crossed, has been developed, and exotic germplasms appearing in each of azaleas are associated whereby new horticultural lines have been created. However, there is no report that a method for breeding a line (individual) having new germ plasmas such as heat resistance and ever-flowering properties is created (Non-Patent Document 1: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991).

Many species of R. dilatatum which flower at early spring the herald of other azaleas come into flower before or substantially the same as development of leaf and, thus, they have very beautiful appears at the flowering period. This is the most feature of R. dilatatum. They also have features other than other azaleas that three leaves having near diamond-shape are verticilated at the edge of branch and that part of R. dilatatum exhibits a laurel property (Non-Patent Documents 4: Teruo Takeuchi "Mitsuba Tsutsuji to Sakura Tsutsuji", Shakunage to Tsutuji, Seibundo Shinko Sya May 1969, p102-120; and Non-Patent Document 5: akashi Yamasaki, and 2 others, Mitsuba Tsutsuji no Syurui to Saibai", Tsutsuji sono Syurui to Saibai, Seibundo Shinko Sya, March 1976, P60-76). It has been said that evergreen R. dilatatum can be bred by crossing deciduous R. dilatatum with evergreen R. eriocarpum, there is no repot, which reports actual breeding (Non-Patent Document 6:).

Kurume azalea is horticultural species which have been crossbred based on R. obtusum and R. sataense and at presnt about 300 species have been widely cultured. Since Kurume azalea blooms a large number of small flowers, there are many species of double flower (hose-in-hose), which is flower type difficult to be scattered, it is popular for potted plant and for garden plant. Its flowering period is from the middle of April to early May, and Kurume azalea has feature of blooming a large number of flowers all together, it becomes essential for cultivation at a pleasure resort (Non-Patent Document 7: Teruo Tamura and 8 others "Kurume Tsutsuji no Saibai to Yurai", Kurume no Tsutsuj i, Ashi Shobo, April 1989, p98-99).

Kurume azalea has been crossbred aiming at flower type, flower color or figure of tree. Since most species of Kurume azalea are selected under an appropriate conditions which ensure eager growth, there arises a problem that the bred azalea cannot withdraw poor conditions. Especially, this is the reason why Kurume azalea at a park on suddenly dies on a park and a pleasure resort of the warm place or green belts along a road (Non-Patent Document 7: Teruo Tamura and 8 others "Kurume Tsutsuji no Saibai to Yurai", Kurume no Tsutsuji, Ashi Shobo, April 1989, p98-99).

As for R. eriocarpum having better wet resistance and heat resistance, since Satsuma archipelago, particularly Tokara islands on which R. eriocarpum grew wild is composed of volcano islands, R. eriocarpum is strong species having resistance to sulfur dioxide. In addition, as indicated by its name, R. eriocarpum has round, small leaves (Non-Patent Document 8: Masaharu Kunishige, "Sonotano Jouryoku Tsutsuji", Tsutsuji sono Syurui to Saibai, Seibundo Shinko Sya, March 1976, P98-99).

Hirado azalea is a species of azalea selected from natural crossing individuals between R. scabrum or R. simsii, which is foreign specie, and R. macrosepalum or R. ripense, which is Japanese specie, around Hirado-shi, Nagasaki-ken, Japan. Some species of this azalea have the biggest tree figure, leaves, and flowers. The tree figure thereof is strong and Hirado azalea grows in a very well manner. Since it has resistance to dry, exhaust gas and sea breeze, it is cultivated everywhere in Japan on a large scale. Hirado azalea has big flower, is rich in flower color, and can keep for long time; thus has merits over other evergreen azaleas (Non-Patent Document 9: Non-Patent Document 9: Teruo Tamura and 8 others, "Hirado Tsutsuji no Saibai to Yurai", Kurume no Tsutsuji, Ashi Shobo, April 1989, p153-162).

Most of azaleas have a one season flowering property which bloom from early spring to summer or autumn, but some of azaleas are of perpetual property which bloom at spring and again bloom at from summer to autumn. Only one line, R. oldhamii, which grow wild on from southeast part of China to Taiwan. This azalea has indumeritum on its leaves, and its flower is only one color, cinnabar, making it poor in enjoyment. However, annual blooming is exhibited, that after blowing at a spring season, flower bud formed after new treetop is not diapaused but blooms (Non-Patent Document 10: Ken-ichi Arisumi and 2 others "Shikizaki Tsutsuji no Ikusyu nikannsuru Kenkyu", Abstract of Presentation of Results of Study in Horticultural Society, 1979, Spring Meeting 1979, p256-257). In general, after blooming at spring season, azalea makes an extension of axillary bud immediately after flower cluster to make floral differentiation, and flower bud is completed up to early autumn. Thereafter, the flower but is dormant due to low temperature, and according to increase in the temperature at the next spring, the flower bud is developed to be flowered. In very restricted one line of R. oldhamii, after starting floral differentiation at a spring season, the flower bud is rapidly complete, which grows without dormancy, and which is then flowered as is. Under strict conditions such as an open field or a glass chamber, it start flowering at a period of from July to August, and in some cases, it keeps on flowering up to the time of frost in an open field, and beyond the year (Non-Patent Document 10: Ken-ichi Arisumi and 2 others "Shikizaki Tsutsuji no Ikusyu nikannsuru Kenkyu", Abstract of Presentation of Results of Study in Horticultural Society, 1979, Spring Meeting 1979, p256-257).

In azaleas, under natural conditions, the flower bud is differentiated at a high temperature period from the last ten day of June to the middle ten days of August (at a temperature of from 18 to 25 deree C) , and from November to October at which the flower bud is completed, azaleas enter into dormant period with decreasing of the temperature. The flower bud dormancy is overcome by encountering a low temperature at winter (from 4 to 7 degree C over a period of from 30 to 40 days), flowering with increasing of temperature at the next spring (Non-Patent Document 11: Masanori Goi, "Tsutsuji No Kaikatokusei"; Shinkaki, No106, June 1980, p72-75)

Japanese Patent Laid-Open Publication 11-266728 (Patent Document 1) discloses a meted for largely propagating plants by forming multi-bud from tissue pieces of plants belonging from genus azalea (particularly column 0005 to 0020). "There is a disclosure that it has been discovered that a starting material which can collect a large number of flower bud, leaf pieces and the like from one parent plant, via multi-bud having a large number of shoots to propagate a large scale of plants belonging to genus azalea with good efficiency, entering the invention."

U.S. Patent 13073 (Patent Document 2) discloses a scarlet as azalea plant. It is disclosed that in novel variety "scarlet", its evergreen leaves are never falling even in a cold season and at a forced cultivation, and that very deep red and semi-double petal flowers are formed, frill flowers are bloomed, its tree is long life and according to infection experiment with Cylindroclaudium, it is low infection ratio.

U.S. Patent 20040073980 (Patent Document 3) discloses "Rosenter 48" as rootstock for Rhododendron. It is also disclosed "Rosenter 48" is new rootstock for Rhododendron which exhibits strong and firm.

Chinese Patent 1413435 discloses a method for producing a seed for Rhododendron delavayi. It discloses a method for regenerating and producing specie of azalea wherein a pool for seed regeneration is constructed, into which a mixture of azalea seed, humans oil and gardening soil (1/70-90/5-15) is prepared, and which is a method for giving regeneration rate of not less than 70% with a low cost.
Patent Document 1 Japanese Patent Laid-Open Publication 11-266728 (column 0005 to column 0020, Fig. 1)
Patent Document 2 U.S. Patent 13073
Patent Document 3 U.S. Patent Publication 20040073980
Patent Document 4 Chinese Patent 1413435
Non-Patent Document 1: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991 Non-Patent Document 2: Kennichi Arisumi "Tsutsuji/Shakunage niokeru Taisho-sei/shinkashoku ryouiki no sosei ni kansuru Kenkyu", Report for Results of Research of subsidy for scientific research expense 1990 (general research C) Them 6356032, March, p1-2, 1991, FIG. 4
Non-Patent Document 3: Kennichi Arisumi, "Taishosei Shakunage no Ikushu (3)", Rhododendron 2003, Vol. 3, No. 1, p67-83 Non-Patent Document 4: Teruo Takeuchi "Mitsuba Tsutsuji to Sakura Tsutsuji", Shakunage to Tsutuji, Seibundo Shinko Sya May 1969, p102-120)
Non-Patent Document 5: Takashi Yamasaki, and 2 others, Mitsuba Tsutsuji no Syurui to Saibai", Tsutsuji sono Syurui to Saibai, Seibundo Shinko Sya, March 1976, P60-76
Non-Patent Document 6: Toshiyuki Goto, "Mitsuba Tsutsuji rui no Ikusyu", Shin Kaki, No106, June 1980, p.45
Non-Patent Document 7: Teruo Tamura and 8 others "Kurume Tsutsuji no Saibai to Yurai", Kurume no Tsutsuji, Ashi Shobo, April 1989, p98-99
Non-Patent Document 8: Masaharu Kunishige, "Sonotano Jouryoku Tsutsuji", Tsutsuji sono Syurui to Saibai, Seibundo Shinko Sya, March 1976, P98-99
Non-Patent Document 9: Teruo Tamura and 8 others, "Hirado Tsutsuji no Saibai to Yurai", Kurume no Tsutsuji, Ashi Shobo, April 1989, p153-162
Non-Patent Document 10: Ken-ichi Arisumi and 2 others "Shikizaki Tsutsuji no Ikusyu nikannsuru Kenkyu", Abstract of Presentation of Results of Study in Horticultural Society; 1979, Spring Meeting 1979, p256-257
Non-Patent Document 11: Masanori Goi, "Tsutsuji No Kaikatokusei", Shinkaki, No106, June 1980, p72-75

### Summary of the Invention

### Problems to be solved by the Invention

However, R. dilatatum is deciduous, and has a problem leaves cannot enjoy throughout year without falling leaves. Kurume azalea cannot withstand poor environments and thus, has a problem that it dies within several years even when it is planted on park or pleasure resort at a warm place or a green belt along a road. In the case of potted azalea, if the flower bud which has been differentiated and developed by a treatment for enlarged day length and a treatment with growth regulator can be overcome dormancy whereby blooming is promoted, it can come into flower at a winter season (one season flowering). However, such a method is applicable to azalea planted on an open field only with very difficulty, and thus, the azalea planted on an open field cannot be ever-flowering.

Based on the discovery that evergreen R. dilatatum, Kurume azalea having applicability to poor environment, and ever-flowering Kurume azalea, which are required from market and which has not yet existed, are bred, the present invention is to provide a method for breeding azalea which introduce into deciduous azalea a gene concerning evergreen, a method for breeding azalea which introduces into one season flowering azalea a gene concerning ever-flowering, and a method for breeding azalea which introduces into non-heat resistant azalea a gene concerning heat resistance, and to provide evergreen R. dilatatum, heat resistant Kurume azalea, and ever-flowering Hirado azalea.

### Means for Solving Problem

In order to solve the problems described above, we has discovered that when deciduous azalea and evergreen azalea are crossed to thereby introduce a gene concerning evergreen possessed by the evergreen azalea into the deciduous azalea, as a result, evergreen inheritance has been succeeded.

Specifically, the present invention is a method for breeding evergreen azalea which breeds deciduous azalea with converting it into an evergreen property, in which a gene concerning evergreen is introduced into the deciduous azalea.

In order to solve the problems described above, we has also discovered that when non-heat resistant azalea and heat resistant azalea are crossed to thereby introduce a gene concerning heat resistance possessed by the heat resistant azalea into the non-heat resistant azalea, as a result; heat resistant inheritance has been succeeded.

Specifically, the present invention is a method for breeding heat resistant azalea which breeds non-heat resistant azalea with converting it into heat resistance, in which a gene concerning heat resistance is introduced into the non-heat resistant azalea.

In order to solve the problems described above, we has also discovered that when one season flowering azalea and ever-flowering azalea are crossed to thereby introduce a gene concerning ever-flowering property possessed by the ever-flowering azalea into the one season flowering azalea, as a result, ever-flowering inheritance has been succeeded.

Specifically, the present invention is a method for breeding ever-flowering azalea which breeds one season flowering azalea with converting it into ever flowering property, in which a gene concerning ever-flowering property is introduced into the one season flowering azalea, the gene which can repeat ever-flowering property every years preferably being introduced into one season flowering azalea.

### Brief Description of the Drawings

FIG. 1 is a drawing showing an incidence of crossed F₁ R. dilatatum according to the present invention. The examination was conducted on February 2002.
FIG. 2 is a drawing showing flower color distribution of Rhododendron weyrichii(⊚, double circle) x R. dilatatum var. satsumense(•) and F₁ hybrid (o) on CIELab standard colorimetric system.
FIG. 3 is a drawing showing flower color distribution of Rhododendron weyrichii(⊚, double circle) x R. reticulatum (•) and F₁ hybrid (o) on CIELab standard colorimetric system.
FIG. 4 is a drawing showing flower color distribution of Rhododendron weyrichii(⊚, double circle) x R. eriocarpum : R. eriocarpum and F₁ hybrid (o) on CIELab standard colorimetric system.
FIG. 5 is a drawing showing an incidence of doube flower (hose-in-hose) in crossed F₁ eriocarpum x Kurume Azalea. The examination was conducted on May 2002.
FIG. 6 is a drawing showing flower color distriution of R. eriocarpum (•) x Kurume Azalea "Imashoujou" (cinnabar red: ⊚, double circle) "and F₁ hybrid (o) on CIELab standard colorimetric system.
FIG. 7 is a drawing showing flower color distriution of R. eriocarpum (•) x Kurume Azalea "Miyagino"(red purple: ⊚, double circle)" and F₁ hybrid (o) on CIELab standard colorimetric system.
FIG. 8 is a drawing showing flower color distriution of R. eriocarpum (•) x Kurume Azalea "Susogo no Ito" (purple: ⊚, double circle)"and F₁ hybrid (o) on CIELab standard colorimetric system.
FIG. 9 is a drawing showing flower color distriution of R. eriocarpum (•) x Kurume Azalea "Kirin " (pink: ⊚, double circle) "and F₁ hybrid (o) on CIELab standard colorimetric system.
FIG. 10 is a drawing showing flower color distriution of R. eriocarpum (•) x Kurume Azalea "Kure No Yuki " (white: ⊚, double circle) "and F₁ hybrid (o) on CIELab standard colorimetric system.
FIG. 11 is a drawing showing monthly change in flowering individuals of Hirado azalea F₁ hybrid. The examination was conducted from November 2001 to January 2003.
FIG. 12 is a drawing showing monthly change in flowering individuals of Hirado azalea F₁ hybrid. The examination was conducted from September 2001 to June 2004.
FIG. 13 is a drawing showing flower color distriution of R. oldhamii(⊚, double circle) x Hirado azalea (Oomurasaki: (•) on CIELab standard colorimetric system.
FIG. 14 is a drawing showing flower color distriution of R. oldhamii (⊚, double circle) x Hirado azalea (Akebono: (•) on CIELab standard colorimetric system.
FIG. 15 is a drawing showing flower color distriution of R. oldhamii (⊚, double circle) x Hirado azalea (Shirotae: (•) on CIELab standard colorimetric system.
FIG. 16 is a drawing showing flower color distriution of R. oldhamii (⊚, double circle) x Hirado azalea (Shounoshin: (•) on CIELab standard colorimetric system.
FIG. 17 is a drawing showing flower color distriution of R. oldhamii (⊚, double circle) x Kurume Azalea, . Large Flower (Miyonosakae (•) on CIELab standard colorimetric system. Best Mode for Carrying Out the Invention

Embodiments of the present invention will now be described. (Impartation of Evergreen Property)

A method for breeding evergreen azalea according to the present invention is converting deciduous azalea into evergreen azalea, and is to introduce a gene concerning evergreen into the deciduous azalea. The term "evergreen azalea" used herein includes azaleas having an inheritance whose leaves are not falling and which annually have leaves.

Examples of deciduous azaleas which can be used in the present invention include, but are not restricted to, Ilam Azalea, R. austrinum, R. pentaphyllum var. nikoense, R. pentaphyllum var. shikokianum, R. atlanticum, R. amagianum, R. amakusaense, R. alabamensis, R. arborescense, R. dilatatum var. decandrum f. lasiocarpum, R. osuzuyamense, Exbury Azalea, R. camtschaticum, R. nipponicum, R. occidentale, Occidental Azalea, R.weyrichii, R. canadense, R. canescens, R. calendulaceum, R. kiyosumense, R. japonicum f. flavum, R. schlippenbachii, R. dilatatum var. lasiocarpum, R. mucronulatum, Ghent Azalea, R. reticulatum, R. nudipes, R. sanctum var. lasiogynum, R. quinquefolium, R. sanctum, R. speciosum, R. wadanum var. lagopus, R. viscistylum, R. weyrichii var. psilostylum, R. yedoense var. hallaisanense, R. wadanum, R. decandrum, Knap Hill Azalea, R. mayebarae, R. nudiflorum, R. semibarbatum, R. dilatatum var. satsumense, R. bakeri, R. hidakanum, R.nagasakianum, R. hyugaense, R. farrerae, R. prunifolium, R. dilatatum, R. tatuoi, R. albrechtii, Mollis Azalea, R. molle, R. yakumontanum, R. lagopus var. niphophilum, Rustica Flore Pleno Azalea, R. luteum, R. japonicum, R. roseum, R. vaseyi, and horticultural vareties and crossed hybrids having parents thereof.

Examples of deciduous azaleas which can be used in the present invention include, but are not restricted to, R.obtusum var. kaempferi f. zonale, Azalea, R. komiyamae, R. amamiense, R. enomotoi, R. serpyllifolium, R. dauricum, R. obtusum var. kaempferi f. latesepalum, R. tschonoskii var. trinerve, R. obtusum var. macrogemma, R. transiens, R. hortense, R. boninense, R. otakumi, R. ripense, R. obtusum var. obtusum, R. obtusum var. kaempferi f. kinshibe, R. oldhamii, Kurume Azalea, R. eriocarpum var. lasiophyllum, R. scabrum, R. tschonoskii, R. obtusum var. kaempferi f. micranthum, R.lapponicum, R. amanoi, R. eriocarpum, R. sataense, R. indicum, R. komatsui, R. obtusumvar. kaempferi f. semperflorens, R. obtusum var. kaempferi f. angustisectum, R. macrosepalum f. leucanthum, R. obtusum var. kaempferi f. album, R. mucronatum, R. latoucheae, R. simsii, R. obtusum var. kaempferi f. tachisene, R. tetramerum, R. yedoense var. poukhanense, R. lusidusculum, R. uwaense, R. nakaharai, R. obtusum var. kaempferi f. multicolor, R. obtusum var. kaempferi f. hananoen, R. hannoense, R. obtusum var. kaempferi f. semiflenum, R. keiskei, Hirado Azalea, R. obtusum var. kaempferi f. latifolium, R. obtusum var. kaempferi f. tubiflorum, R. tosaense, R. macrotransiens, R. obtusum var. kaempferi f. angustifolium, R. eriocarpum, R. obtusum var. mikawanum, R. tectum, R. kiusianum, R. obtusum var. kaempferi f. mikawanum, R. macrosepalum, R. obtusum var. kaempferi f. komatsui, R. yakuinsulare, R. obtusum var. kaempferi, and horticultural vareties and crossed hybrids having parents thereof.

As described above, in the present invention, a gene of evergreen azalea is introduced into deciduous azalea, and an evergreen azalea as a flower parent or seed parent can be crossed with deciduous azalea to introduce a gene of evergreen azalea into deciduous azalea. For example, R. reticulatum (Rhododendron reticulatum), R. dilatatum var. satsumense (Rhododendron satsumense) or Rhododendron weyrichii as deciduous azalea is crossed with R. eriocarpum (Rhododendron eriocarpum) as evergreen azalea. Furthermore, in the present invention, crossing with flower parent or seed parent of evergreen azalea may be mutual crossing. For example, deciduous Rhododendron weyrichii may be crossed with deciduous Rhododendron satsumense to breed evergreen azalea.

These azaleas may be suitably selected depending upon types of flowers (color, flower type etc.), but it is preferable to select azaleas based on our non-disclosed Patent Application PCT/JP2004/000297(Japanese Patent Application 2003-144406", entitled "Method For Crossing Flowering Plants Based on Their Pigment Genotypes".

Specifically, according to this application, there is description that "the present invention is based on the discovery of new inheritance law from flower pigment phenotypes of main flower pigments F1 to F4 generations that as a result of examination of self-pollination and cross pollination taking account of inheritance of three anthocyanidins, pelargonidin (Pgn), cyanidin (Cyn), and delphinidin (Dpn), which are main flower pigments. Also, with regard to Pgn phenotype and Dpn phenotype, it has been discovered that Pgn and Dpn pigments are not co-existing, but they are existing as sole types, or they are inherited together with Cyn pigment". It is also disclosed that "Flower petal pigment of azalea (Ericaceae) was analyzed to examine pigment genotypes of flower petal of various lines", "R. oldhamii was used as seed parent and Hirado azalea was used as pollen parent to make a crossing to create ,F1 azalea", that "Kurume azalea which is hose-in-hose flower, and R. eriocarpum, which is single flower azalea were crossed to separate 144 individuals of hose-in-hose flower hybrid and 123 individuals of single flower hybrid at a ratio of 1:1. As a result, it has been clarified that genotype of Kurume azalea which is hose-in-hose flower and that of hose-in-hose flower hybrid were Dₕd (hetero) and the genotype of Satsuki, which is single flower azalea and that of single flower hybrid were dd (recessive homozygote)."

In the present invention, it is clear that the kind of flower and type of flower are predicted to select the plants from such genotypes.

### (Introduction of Heat Resistance)

In another embodiment of the present invention, alternative to or in addition to breeding evergreen azalea from deciduous azalea and evergreen azalea, it is possible to breed heat resistant azalea from non-heat resistant azalea and heat resistant azalea.

The term "heat resistant azalea is azalea having inheritance that it can grow even under a high temperature and high humidity (herein after referred to as "heat resistant inheritance"). The heat resistant azalea is not specifically restricted as long as such a heat resistant inheritance. For example, amongst deciduous azaleas described above, those having heat resistance are Rhododendron weyrichii and R. dilatatum var. satsumense and others are non-heat resistant, deciduous azaleas. On the other hand, amongst evergreen azaleas described above, those having heat resistance are R. scabrum, R. sataense, Hirado azalea, R. eriocarpum, and R. obtusum and others are non-heat resistant evergreen azaleas.

Selection of heat resistant azalea and non-heat resistant azalea may be suitably conducted depending upon types of flowers (color, flower type etc.) as in the case of introduction of evergreen property.

In the present invention, a heat resistant gene is introduced into non-heat resistant azalea to make it possible to breed heat resistant azalea.

It is noted that when the azalea having evergreen property imparted thereto according to the present invention is used as one of non-heat resistant azalea or heat resistant azalea, the selected range of azalea for crossing is widened and, azalea having evergreen property and heat resistance at the same time can be bred.

### (Introduction of Ever-Flowering Property)

In still another embodiment of the present invention, alternative to or in addition to breeding evergreen azalea from deciduous azalea and evergreen azalea and/or breeding heat resistant azalea from non-heat resistant azalea and heat resistant azalea, it is possible to breed ever-flowering azalea from one season flowering azalea and ever-flowering azalea.

The term "ever flowering azalea" used herein is intended to azalea having annually flowering inheritance (herein after referred to as "ever flowering inheritance", and one season flowering azaleas having no ever flowering inheritance.

In the present invention, the ever-flowering azalea is not specifically restricted as long as it has ever flowering inheritance, and an example includes R. oldhamii amongst deciduous azaleas and evergreen azaleas described above. On the other hand, one season flowering azaleas which can be mentioned are those of deciduous azaleas and evergreen azaleas described above except for R. oldhamii.

As descried above, crossing of one season flowering azalea with ever-flowering azalea can make it possible to impart an azalea having characteristics of one season flowering azalea to ever-flowering azalea.

Similar to introduction of evergreen property, one season flowering azalea may be suitably conducted depending upon types of flowers (color, flower type etc.).

Furthermore, it is within the scope of the present invention that crossed hybrid obtained by crossing ever-flowering azalea with one season flowering azalea is used as ever-flowering azalea. Similarly, a crossed variety having evergreen inheritance, heat resistant inheritance, or both inheritances can also be suitably used as a parent plant.

It is noted that when the azalea having evergreen property imparted thereto or the azalea having evergreen property and heat resistance imparted thereto according to the present invention is used as one of one season flowering azalea or ever-flowering azalea, the selected range of azalea for crossing is widened and, ever-flowering azalea having evergreen property and heat resistance at the same time can be bred.

### [Example 1]

Specific methods for breeding azalea according to the present invention will be described based on Examples.

### [How to Store Pollen]

Anther from which azalea pollen was collected small flower was packed into paper for wrapping individual doses of powdered medicine, was introduced a glass bin having silica gel contained therein, and was dried for three days. Thereafter, the anther was stored in a refrigerator at a temperature of -20 degree C to be ready for use. The crossing was carried out within a glass chamber which was not warmed. First petal and stamen were removed, and after two or three days, it was confirmed mucilage was sufficiently secreted from the edge of the stamen, and the pollen which had been stored was applied to the stigma.

### [Example 2]

### [How to Store Crossed Seed]

Five to six months after azaleas were crossed, capsules which had been sufficiently matured and which epicarp became brown, were collected, dried at a room temperature to be cleaved. Subsequently, the resulting seeds were packed into paper for wrapping individual doses of powdered medicine, they were introduced into a glass bin having silica gel contained therein, and stored at a room temperature until seeding.

### [Example 3]

### [How to Seed Crossed Seed: No. 1: Seeding on Jiffy Pot]

Crossed seeds of azalea were seeded according to the following method. A soil prepared by mixing fine granules of Akadama soil, fine granules of Kanuma soil, and fine granules of Bora soil in a proportion of 1:1:1 was poured into a Jiffy pot (having 8.5 cm in a diameter) to seven-tenth at a first of December, and approximately 0. 5 cm of finely divided sphagnum was placed thereon, on which the crossed seeds were seeded. The soil was previously conducted sterilization and destroying pests with a 1000 times diluted liquid of Danicoal and with a 1000 times diluted liquid of Sumithione. Number of seeding was 100 seeds per one pot, and water was well sprinkled after seeding, and pots were transferred within frames covered with transparent vinyl kept at 25 degree C installed within a glass chamber. Germination was observed approximately 20 days after seeding. From immediately after germination to the last ten days of the last March, a fluorescent lamp for plant was used for lighting 4 hours at midnight (from 10: 00 p.m. to 2:00 a.m.) for light supplement. After germination, a liquid fertilizer from fermented oil cake whose N: P: K was formulated to 4:4:3 was given at 1000 time dilution also for water sprinkling per week.

### [Example 4]

### [How to Seed Crossed Seed: No. 2: Seeding with Cell Tray]

Crossed seeds of azalea were seeded according to the following method. A soil prepared by mixing fine granules of Akadama soil, fine granules of Kanuma soil, and fine granules of Bora soil in a proportion of 1:1:1 was poured into a 128 well cell tray (having a size of 54 x 28 x 5 cm) to eight-tenth at a first of December, and finely divided sphagnum was placed thereon, on which the crossed seeds were seeded. Number of seeding was from 5 to seven seeds per one well. Water was well sprinkled after seeding, and the cell tray was transferred within frames covered with transparent vinyl kept at 25 degree C installed within a glass chamber. From immediately after germination to the last ten days of the last March; a fluorescent lamp for plant was used for lighting 4 hours at midnight (from 10:00 p.m. to 2:00 a.m.) for light supplement. After germination, a liquid fertilizer from fermented oil cake whose N: P: K was formulated to 4:4:3 was given at 1000 time dilution also for water sprinkling per week.

Up to the last April, seeds was thinned out so as to one or two individuals per one well of cell tray. This was transferred to an incubator kept at a high temperature of 32 degree C, and this was bred for three months up to the first ten days of July at an illuminance of 3000 lux for 16 hours of day length. During this course, 0. 1% Hyponex soluton (trade name of Kabushiki Kaisya Hyponex Japan) was given per two weeks, and 1000 times diluted liquid of Danicoal (trade name EDS Kabushiki Kaisha) and with a 1500 times diluted liquid of Sumithione (trade name: Kyushu Sankyo Kabushiki Kaisha) were sprinkled. The cell tray was transferred to a glass chamber not warmed at the first ten days of July, and a liquid fertilizer from fermented oil was given per two weeks at 1000 time dilution, and 1000 times diluted liquid of Danicoal (trade name EDS Kabushiki Kaisha) was sprinkled per two weeks.

### [Example 5]

### [Plantation of Seedling]

First plantation of seedling according method No. 1 was conducted from the last ten days of the next March to the first ten days of the next April. The crossed seedling extending to 0.5 cm to 1 cm was planted on a plant bed into which soil prepared by mixing fine granules of Akadama soil, fine granules of Kanuma soil, and fine granules of Bora soil in a proportion of 1:1:1 was poured eighth-ten. Number of plantation was from 120 to 140 individuals per one plant bed.

### [Example 6]

### [How to Manage Breeding after Plantation of Seedling: No. 1]

After plantation of the seedlings, they were transferred to a glass chamber not warmed. As for management after plamtation, a liquid fertilizer from fermented oil cake whose N:P:K was formulated to 4:4:3 was given at 1000 time dilution also for water sprinkling per two weeks. In order to avoid stand blighting of the seedling, a liquid fertilizer from fermented oil was given per two weeks at 1000 time dilution, and 1000 times diluted liquid of Danicoal(trade name EDS Kabushiki Kaisha) was sprinkled per two weeks.

At the last ten weeks of March next the first plantation, second plantation was conducted. One crossed seedlings living on the plant bed was planted on one black vinyl-made pod having a diameter of 9 cm into which soil prepared by mixing fine granules of Akadama soil, fine granules of Kanuma soil, and fine granules of Bora soil in a proportion of 1:1:1 was poured eighth-ten.

Furthermore, at the last ten weeks of March next the second plantation, third plantation was carried out on a black vinyl-made pod having a diameter of 15 cm with the same soil. As for the breeding management from the second plantation to 4 years, oil cake was given as a set fertilizer per three months, and a 1500 times diluted liquid of Sumithione (trade name: Kyushu Sankyo Kabushiki Kaisha) were sprinkled per one month.

### [Example 7]

### [How to Manage Breeding after Plantation of Seedling: No. 2]

After plantation of seedling, the seedlings were transferred to an incubator kept at a high temperature of 32 degree C, and they were bred for three months up to June 30 at an illuminance of 3000 lux for 16 hours of day length. During this high temperature treatment, 0.1% Hyponex soluton (trade name of Kabushiki Kaisya Hyponex Japan) was given per two weeks, and 1000 times diluted liquid of Danicoal (trade name EDS Kabushiki Kaisha) and with a 1500 times diluted liquid of Sumithione (trade name: Kyushu Sankyo Kabushiki Kaisha) were sprinkled. At July 1, the plant bed was transferred to a glass chamber not warmed, and a liquid fertilizer from fermented oil was given per two weeks at 1000 time dilution, and 1000 times diluted liquid of Danicoal(trade name EDS Kabushiki Kaisha) was sprinkled per two weeks.

At the last ten weeks of March next the first plantation, second plantation was conducted. One crossed seedlings living on the plant bed was planted on one black vinyl-made pod having a diameter of 9 cm into which soil prepared by mixing fine granules of Akadama soil, fine granules of Kanuma soil, and fine granules of Bora soil in a proportion of 1:1:1 was poured eighth-ten. During this course, from May of plantation oil, cake was given as a set fertilizer per three months, and a 1500 times diluted liquid of Sumithione (trade name: Kyushu Sankyo Kabushiki Kaisha) were sprinkled per one month.

### [Example 8]

From the last ten days of March 1997 to the middle ten days of April 1997, the crossing was conducted as four combinations described below.
(1): Rhododendron weyrichii (from Amakusa, Kumamoto, potted) x R. dilatatum var. satsumense(Rock Azalea from Makisono-cho, Kagoshima, potted)
(2): Rhododendron weyrichii (from Amakusa, Kumamoto, potted) x R. reticulatum (from Fusayama, Kumamoto, potted)
(3): Rhododendron weyrichii (from Amakusa, Kumamoto, potted) x R. eriocarpum(Yakushima, Kagoshima, potted)
(4): R. dilatatum (purple red, potted) x R. dilatatum var. satsumense (Rock Azalea from Makisono-cho, Kagoshima, potted)

Number of crossed flowers, bolling number (rate), total seed number, seeding number, and germination rate were examined. The results are shown in Table 1. In the case of crossing of species each having different flowering period, one which has faster flowering period was used as a pollen parent, and one which has latter flowering period was used as a seed parent. The date of crossing was from March 27, 1997 to April 11 1997, the date of examination was the middle ten days of October 1997, the date of bolling was the last ten days of November 1997, the date of seeding was from December 10 to 12, 1997, and the date of examination of germination was from March 26, 1998 to April 3, 1998.

The change in survival rate was examined. Table 2 and Table 3 show change in survival of seedling after first and second plantation. In the first plantation, 120 seedlings were planted per one plant bed, and in the second plantation, each one individuals were planted on a vinyl-inade pot. The survival rate in Table 2 described within parentheses was expressed by taking the number of plantation planted on April 1, 1998 as 100%. The date of plantation was from March 26, 1998 to April 3, 1998 (First Plantation), and the date of examining survival rate was approximately the last date of the examination month as shown in Table 2. The survival rate in Table 3 described within parentheses was expressed by taking the number of plantation planted on April 1, 1999 as 100%. The date of plantation was from March 28, 1999 to April 1, 1999 (Second Plantation), and the date of examining survival rate was approximately the last date of the examination month as shown in Table 3.

**Table 1**

| Combination of Crossing | No. of Crossing | Bolling No.(%) | Total Seed No. | Seeding No.(%) | Germination Rate(%) |
|---|---|---|---|---|---|
| R.weyrichii x R. satsumense | 24 | 22 (91.7) | 4840 | 2300 | 64.1 |
| R.weyrichii x R. reticulatum | 25 | 19 (76.8) | 6270 | 4535 | 46.5 |
| R.weyrichii x R. eriocarpum | 22 | 13 (59.1) | 5550 | 5100 | 37.7 |
| R.dilatatum x R. satsumense | 27 | 14 (51.9) | 2820 | 2200 | 48.2 |

In any combination of the four combinations of crossing shown in Table 1, the bolling rate was from approximately 50 to 90%, imcompatibility of crossing was not observed. As for the germination rate, although the combination of crossing Rhododendron weyrichii x R. eriocarpum was 37.7%, which was relatively low, the value of germination rates of other three combinations were from approximately 47% to 67%. In particular, the combination of crossing Rhododendron weyrichii x R. dilatatum var. satsumense showed relatively high germination rate, which was 64%.

**Table 2**

| Line | Planting No. (Apr.) | Survival No. (rate) | | | | | |
|---|---|---|---|---|---|---|---|
| | | May | Jun. | Sept. | Nov. | Jan. | Mar. |
| R.weyrichii x R. satsumense | 480 | 442 (92) | 408 (85) | 384 (80) | 379 (79) | 374 (78) | 350 (73) |
| R.weyrichii x R. reticulatum | 480 | 422 (88) | 350 (73) | 283 (59) | 240 (50) | 240 (50) | 224 (47) |
| R.weyrichii x R. eriocarpum | 480 | 451 (94) | 312 (65) | 278 (58) | 230 (48) | 216 (45) | 206 (43) |
| R.dilatatum x R. satsumense | 480 | 360 (75) | 245 (66) | 245 (51) | 202 (42) | 192 (40) | 148 (31) |

**Table 3**

| Line | Planting No. (Apr.) | Survival No. (rate) | | | | | | |
|---|---|---|---|---|---|---|---|---|
| | | Jun. | Sept. | Dec. | Mar. | Jun. | Sept. | Dec. |
| R.weyrichii × R. satsumense | 286 | 214 (75) | 199 (70) | 197 (69) | 195 (68) | 193 (67) | 191 (67) | 191 (67) |
| R.weyrichii x R. reticulatum | 174 | 121 (70) | 85 (49) | 82 (47) | 81 79 (47) | (45) | 77 (44) | 77 (44) |
| R.weyrichii x R. eriocarpum | 187 | 148 (79) | 110 (59) | 109 (58) | 107 (57) | 105 (56) | 104 (56) | 103 (55) |
| R.dilatatum x R. satsumense | 102 | 72 (71) | 40 (39) | 39 (38) | 38 (37) | 37 (36) | 35 (34) | 35 (34) |

As can be seen from Table 2 and Table 3, four combinations of crossling after two to three months had high survival rates, which were not less than 70% in both plantations, and at the last date of September, six months after the plantation, the combination of crossing Rhododendron weyrichii x R. dilatatum var. satsumense kept high survival rate. In other combination, the survival rates were somewhat decreased. However, after October, they substantially kept the suvirval rate at the last day of September. Although treatment of plantation and high temperature at a summer season stressed the growth in the seedlings, more than half of seedlings were survived.

### [Example 9]

From the last ten days of March 2000 to the middle ten days of April 2000, the breeding was conducted as six combinations described below.
(1): Rhododendron weyrichii (from Amakusa, Kumamoto, potted) x R. dilatatum var. satsumense(Rock Azalea from Makisono-cho, Kagoshima, potted)
(2)Rhododendron weyrichii from Amakusa, Kumamoto, potted) x R. reticulatum (from Fusayama, Kumamoto, potted)
(3) Rhododendron weyrichii (from Amakusa, Kumamoto, potted) x R. eriocarpum (Yakushima, Kagoshima, potted)
(4) R. dilatatum var. satsumense (Rock Azalea from Makisono-cho, Kagoshima, potted) x R. reticulatum (from Fusayama, Kumamoto, potted)
(5) R. dilatatum var. satsumense (Rock Azalea from Makisono-cho, Kagoshima, potted) x R. eriocarpum (Yakushima, Kagoshima, potted)
(6) R.reticulatum (Fusayama, Kumamoto, potted) x R. eriocarpum (Yakushima, Kagoshima, potted)

Number of crossed flowers, boiling number (rate) , total seed number, seeding number, and germination rate were examined. The results are shown in Table 4. In the case of crossing of species each having different flowering period, one which has faster flowering period was used as a pollen parent, and one which has latter flowering period was used as a seed parent. The crossing of these six combination was conducted from the last ten days of March to the middle ten days of April using the stored pollens.

In Table 4, the date of crossing was from March 31, 2000 to April 22 2000, the date of examination of bolling was the middle ten days of September 2000, the date of collection was the first ten days of November 2000 , the date of seeding was from December 1 to 4, 2000, and the date of examination of germination was from March 29, 2001 to April 1, 2001.

In Table 5, the date of crossing was from March 29, 2001 to April 15 2001, the date of examination of bolling was the last ten days of October 2001, the date of collection was the middle ten days of November 2001 , the date of seeding was from December 5 to 9, 2000, and the date of examination of germination was from April 2, 2002 to April 5, 2002.

The change in survival rate was examined. Table 6 shows change in survival of seedling after first plantation on the plant bed conducted from the last ten days of March 2001 to the first ten days of April 2001 and then high temperature treatment over a period of three months. Also, Table 7 shows change in survival of seedling after second plantation on the vinyl-made pot conducted from the last ten days of March 2001 to the first ten days of April 2001.

The survival rate in Table 6 described within parentheses was expressed by taking the number of plantation planted on April 1, 2001 as 100%. The date of plantation was from March 29, 2001 to April 1, 2001 (First Plantation), and then high temperature treatment over a period of three months. The date of examining survival rate was approximately the last date of the examination month as shown in Table 6. The survival rate in Table 6 described within parentheses was expressed by taking the number of plantation planted on April 1, 2002 as 100%. The date of plantation was from March 26, 2001 to March 29 2001 (Second Plantation), and the date of examining survival rate was approximately the last date of the examination month as shown in Table 7.

**Table 4**

| Combination of Crossing | No. of Crossing | Bolling No.(%) | Total Seed No. | Seeding No.(%) | Germination Rate(%) |
|---|---|---|---|---|---|
| R.weyrichii x R. satsumense | 12 | 10 (83.3) | 4300 | 2640 | 70.9 |
| R.weyrichii x R. reticulatum | 10 | 8 (80.0) | 3360 | 2350 | 62.8 |
| R.weyrichii x R. eriocarpum | 12 | 7 (58.3) | 2660 | 2370 | 38.4 |
| R. satsumense x R. eriocarpum | 16 | 12 (75.0) | 3120 | 2220 | 35.5 |
| R. reticulatum x R. satsumense | 12 | 10 (83.3) | 1500 | 1500 | 57.7 |
| R. reticulatum x R. eriocarpum | 14 | 9 (64.3) | 1350 | 1350 | 32.3 |

**Table 5**

| Combination of Crossing | No. of Crossing | Bolling No.(%) | Total Seed No. | Seeding No.(%) | Germination Rate(%) |
|---|---|---|---|---|---|
| R.weyrichii x R. satsumense | 12 | 11 (88.7) | 3850 | 2560 | 72.5 |
| R.weyrichii x R.weyrichii x R. reticulatum | 12 | 10 (83.3) | 3650 | 2560 | 68.0 |
| R.weyrichii x R. eriocarpum | 12 | 7 (58.3) | 1750 | 1280 | 33.3 |
| R. satsumense x R. eriocarpum | 12 | 6 (50.0) | 1680 | 1280 | 39.8 |
| R. reticulatum x R. satsumense | 12 | 10 (83.3) | 3400 | 2560 | 75.1 |
| R. reticulatum x R. eriocarpum | 12 | 5 (41.7) | 1350 | 1280 | 38.8 |

From the results of Table 4 (crossing on 2000) and Table 5, the bolling rate was from approximately 50 to 90%, except for the combination of crossing R.reticulatum x R. eriocarpum, which was 42%, imcompatibility of crossing was not observed. As for the germination rate, although the combination of crossing utilizing R. eriocarpum as one partent was not more than 40%, which was relatively low, the value of germination rates of other combinations were not less than 58%. In particular, the combination of crossing Rhododendron weyrichii x R. dilatatum var. satsumense, and the combination of R. reticulatum x R. dilatatum var. satsumense in Table 5 showed relatively high germination rate, which was not less than 70%.

**Table 6**

| Line | Planting No. (Apr.) | Survival No. (rate) | | | | | |
|---|---|---|---|---|---|---|---|
| | | May | Jun. | Sept. | Nov. | Jan. | Mar. |
| R.weyrichii x R. satsumense | 960 | 845 (88) | 662 (69) | 547 (57) | 442 (46) | 432 (45) | 413 (43) |
| R.weyrichii x R. reticulatum | 960 | 883 (92) | 720 (75) | 557 (58) | 509 (53) | 490 (51) | 461 (48) |
| R.weyrichii x R. eriocarpum | 960 | 749 (78) | 672 (70) | 586 (61) | 461 (48) | 442 (46) | 442 (46) |
| R. satsumense x R. eriocarpum | 960 | 893 (93) | 710 (74) | 528 (55) | 451 (47) | 413 (43) | 403 (42) |
| R. reticulatum x R. satsumense | 960 | 720 (75) | 595 (62) | 490 (51) | 365 (38) | 336 (35) | 336 (35) |
| R. reticulatum x R. eriocarpum | 960 | 768 (80) | 653 (68) | 528 (55) | 403 (42) | 403 (42) | 384 (40) |

**Table 7**

| Line | Planting No. (Apr.) | Survival No. (rate) | | | |
|---|---|---|---|---|---|
| | | Jun. | Sept. | Dec. | Mar. |
| R.weyrichii x R. satsumense | 200 | 168 (84) | 144 (72) | 128 (64) | 124 (62) |
| R.weyrichii x R. reticulatum | 200 | 164 (82) | 152 (76) | 152 (76) | 148 (74) |
| R.weyrichii x R. eriocarpum | 200 | 156 (78) | 144 (72) | 160 (80) | 130 (65) |
| R. satsumense x R. eriocarpum | 200 | 170 (85) | 160 (80) | 156 (78) | 156 (78) |
| R. reticulatum x R. satsumense | 200 | 168 (84) | 160 (80) | 160 (80) | 152 (76) |
| R. reticulatum x R. eriocarpum | 200 | 176 (88) | 168 (84) | 164 (82) | 164 (82) |

Table 6 shows change in survival of seedling after first plantation on the plant bed conducted from the last ten days of March 2001 to the first ten days of April 2001 and then high temperature treatment over a period of three months. At the last day of May, all combinations of crossing showed survival rate of not less than 75%, but at the last day of September, survival rate was drastically decreased to from 51 to 61%. Thereafter, the survival rate was gradually decreased, and at the last day of March, the survival rate showed 35-48% (Table 6). In contrast, in the case of second plantation on the vinyl-made pot conducted from March 26, 2001 to March 29, 2001 shown in Table 7, the decreasing of the survival rate after the plantation is not so large. At the last day of June 2002, the survival rate of each combination was 78-88%, and at the last day of March 2003, it was only decreased to 62-82% (Table 7).

### [Example 10]

From the last ten days of March 2001 to the middle ten days of April 2000, the breeding was conducted as six combinations described below.
(1): Rhododendron weyrichii (from Amakusa, Kumamoto, potted) x R. dilatatum var. satsumense(Rock Azalea from Makisono-cho, Kagoshima, potted)
(2)Rhododendron weyrichii from Amakusa, Kumamoto, potted) x R. reticulatum (from Fusayama, Kumamoto, potted)
(3) Rhododendron weyrichii (from Amakusa, Kumamoto, potted) x R. eriocarpum (Yakushima, Kagoshima, potted)
(4) R. dilatatum var. satsumense (Rock Azalea from Makisono-cho, Kagoshima, potted) x R. eriocarpum (Yakushima, Kagoshima, potted)
(5) R. reticulatum (Fusayama, Kumamoto, potted) x R. dilatatum var. satsumense (Rock azalea. Makisono-cho, Kagoshima, potted)
(6) R.reticulatum (Fusayama, Kumamoto, potted) x R. eriocarpum (Yakushima, Kagoshima, potted)

With regard to these crossed seeds, no plantation was conducted, but seedlings thereof directly grown on the cell tray were adjusted to be one or two individuals per one well of the cell tray, and the high temperature treatment was conducted over a period of three months from the first ten days of April 2002. The survival rates thereof are shown in Table 8.

The survival rate in Table 8 described within parentheses was expressed by taking the number of seedlings adjusted on April 1, 2001 as 100%. The date of adjusting the seedlings was from April 2, 2002 to April 5, 2002, and after the adjustment, the heat treatment was conducted over a period of three months. The date of examining survival rate was approximately last day of the month of the examination date described in Table 8.

**Table 8**

| Line | Planting No. (Apr.) | Survival No. (rate) | | | | | |
|---|---|---|---|---|---|---|---|
| | | May | Jun. | Sept. | Nov. | Jan. | Mar. |
| R.weyrichii x R. satsumense | 512 | 451 (88) | 440 (86) | 410 (80) | 399 (78) | 389 (76) | 379 (74) |
| R.weyrichii x R. reticulatum | 512 | 471 (92) | 471 (92) | 399 (78) | 379 (74) | 369 (72) | 369 (72) |
| R.weyrichii x R. eriocarpum | 512 | 456 (89) | 440 (86) | 415 (81) | 410 (80) | 410 (80) | 404 (79) |
| R. satsumense x R. eriocarpum | 512 | 451 (88) | 435 (85) | 410 (80) | 394 (77) | 384 (75) | 384 (75) |
| R. reticulatum x R. satsumense | 512 | 492 (96) | 471 (92) | 435 (85) | 399 (78) | 384 (75) | 384 (75) |
| R. reticulatum x R. eriocarpum | 512 | 461 (90) | 430 (84) | 420 (82) | 420 (82) | 415 (81) | 410 (80) |

As a result of Table 8, survival rates in all combination of crossing at the last day of June which showed from 78% to 88%, and survival rates at the last day of the next March showed from 62 to 82%, indicating that the survival rate was not so decrease. From the results, it has been discovered that the survival rate was decreased by plantation, one of causes for decreasing the survival rate was plantation rather than high temperature environment.

### [Example 11]

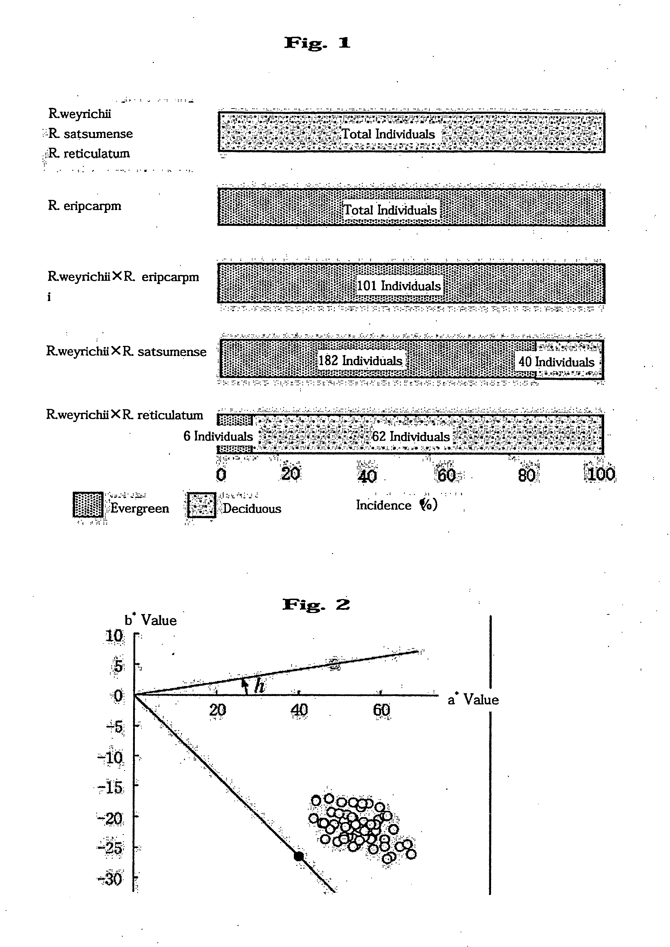

As for F₁ hybrids of 391 individuals survived by crossing the following three combinations conducted from the last ten days of March 1997 to the middle ten days of April 1997, the results of evergreen property (deciduous property at a winter season) are shown in FIG. 1.
(1) Rhododendron weyrichii(Amakusa, Kumamoto, potted) x R. dilatatum var. satsumense (Rock azalea, Makisono-cho, Kagoshima, potted):
(2) Rhododendron weyrichii (Amakusa, Kumamoto, potted) x R. reticulatum (Fusayama, Kumamoto, potted): and
(3) Rhododendron weyrichii(Amakusa, Kumamoto, potted) x R. eriocarpum (Yakushima, Kagoshima, potted).

As shown in FIG. 1, it has been discovered that in the F₁ seedling of crossing Rhododendron weyrichii x R. eriocarpum, all of examined 101 individuals possess evergreen property. Also, it has been discovered that 182 individuals (88%) of 222 individuals in the F₁ seedling of Rhododendron weyrichii x R. dilatatum var. satsumense, and 6 individuals (9%) of 68 individuals in the F₁ seedling of Rhododendron weyrichii x R. reticulatum possess evergreen property. Since the F₁ seedling utilizing R. eriocarpum as one parent had 100% evergreen property, evergreen property possessed by R. eriocarpum : R. eriocarpum was dominant inheritance, and evergreen inheritance was succeeded in crossing progeny utilizing : R. eriocarpum.

### [Example 12]

Flower color of the flower petals of evergreen azalea, which were flowering at March 2003, from the following three combinations of crossing conducted from the last ten days of March 1997 to the middle ten days of April 1997 were examined.
(1) Rhododendron weyrichii (Amakusa, Kumamoto, potted) x R. dilatatum var. satsumense (Rock azalea, Makisono-cho, Kagoshima, potted);
(2) Rhododendron weyrichii (Amakusa, Kumamoto, potted) x R. reticulatum (Fusayama Kumamoto, potted); and
(3) Rhododendron weyrichii (Amakusa, Kumamoto, potted) x R. eriocarpum (Yakushima Kagoshima, potted) . The flower petals were collected, color is measured with a coloriemter to measure L*a*b* value, from which a hue angle (h) was calculated. More specifically, total 101 individuals flowered within March 2003, comprising 58 individuals from the crossing Rhododendron weyrichii x R. dilatatum var. satsumense, 17 individuals from crossing Rhododendron weyrichii x R. reticulatum, 17 individuals from crossing Rhododendron weyrichii x R. eriocarpum, and 9 individuals from R. dilatatum var. satsumense were examined for flower color.

As for crossed seedling of Rhododendron weyrichii x R. dilatatum var. satsumense,the flower petal of Rhododendron weyrichii being crossing parent indicated a* value = 47.9 and b* value =5.4, that of R. dilatatum var. satsumense being a parent indicated a* value =39.8, and b* value =-25.2. As for the seedlings of F₁ crossing, a* value had individual distribution from 43.5 to 68.8, and b* value was individual distribution from -17.2 to -27.5, and the hue angle h showing the flower color (in FIG. 2, taking the crosspoint of a* axis which is a horizontal axis and b* axis which is a vertical axis, i.e., 0 as a center, drawing a linear line toward, for example, point (⊚, double circle) on which Rhododendron weyrichii was plotted, and expressing an angle produced by this line and a* axis, the same as above) showed a distribution within the scope of the crossing parent. The chromaticity distribution of the crossed seedling was clustered toward R.dilatatum var. satsumense rather than Rhododendron weyrichii, indicating that F₁ hybrid has a flower color resembling that of R. dilatatum var. satsumense (FIG. 2).

As for crossed seedling of Rhododendron weyrichii x R. reticulatum, the flower petal of R. reticulatum being one parent indicated a* value = 57.5, and b* value = -23.8. As for the seedlings of F₁ crossing, a* value had individual distribution from 35.2 to 62. 8, and b* value was individual distribution from -13.7 to -23. 8, and as for the hue angle h showing the flower color, there were a distribution beyond the hue angle of : R. reticulatum, and a distribution within the hue angle of the crossing parents. The chromaticity distribution of the crossed seedling was clustered toward R. reticulatum indicating that F₁ hybrid has a flower color resembling that of R. reticulatum.

As for crossed seedling of Rhododendron weyrichii x R. eriocarpum, the flower petal of R. eriocarpum reticulatum being one parent indicated a* value = 4.7, b* value = -2.5. As for the seedlings of F₁ crossing, a* value had individual distribution from 15.2 to 57.5, and b* value was individual distribution from -6.9 to -21.6, and the hue angle h showing the flower color showed a distribution within the scope of the crossing parent. The chromaticity distribution of the crossed seedling was clustered toward R. eriocarpum rather than Rhododendron weyrichii, indicating that F₁ hybrid has a flower color resembling that of R. eriocarpum.

### [Example 13]

From the last ten days of March to the middle ten days of April of 1997, 2000, and 2001, the crossing of the following five combination was conducted.
(1) R. eriocarpum (Nakanoshima Kagoshina, potted) x Kurume Azalea "Imashoujou" (cinnabar red, double flower, open field)
(2) R. eriocarpum (Nakanoshima Kagoshina, potted) x Kurume Azalea "Miyagino" (pruple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Homare No Yuki" (white, double flower, potted).

In any combination of crossing, Kurume azalea, which flowers at the last ten days of April, was used as pollen parent, and R. eriocarpum, which flowers in later at the first ten days of May, was used as a seed parent. Anther from which azalea pollen was collected small flower was packed into paper for wrapping individual doses of powdered medicine, was introduced a glass bin having silica gel contained therein, and was dried for three days. Thereafter, the anther was stored in a refrigerator at a temperature of -20 degree C to be ready for use. The crossing was carried out within a glass chamber which was not warmed. First petal and stamen were removed, and after two or three days, it was confirmed mucilage was sufficiently secreted from the edge of the stamen, and the pollen which had been stored was applied to the stigma. Five to six months after azaleas were crossed, capsules which had been sufficiently matured and which epicarp became brown, were collected, dried at a room temperature to be cleaved. Subsequently, the resulting seeds were packed into paper for wrapping individual doses of powdered medicine, they were introduced into a glass bin having silica gel contained therein, and stored at 4 degree C until seeding.

Seeding was conducted on a Jiffy pot. From March 24 1998 to April 1, 1998, first plantation was conducted, from March 26 to 29, 1998, second plantation was conducted, and from March, 18 to 22, 1999, third plantation was conducted. Number of crossing flower, boiling number (boiling rate), number of seeds, number of seeding, and germination rate were examined. The results are shown in Table 9, Table 10, and Table 11.

In Table 9, the date of crossing was from June 6 to 25, 1997 the date of examination was the last ten days of November 1997, the date of bolling was November 11, 1997, the date of seeding was from December 8 to 10, 1997, and the date of examination of germination was from March 31, 1998 to April 1, 1998.

In Table 10, the date of crossing was from May 31, 2000 to June 19, 2000 the date of examination was the last ten days of November 2000, the date of bolling was November 21, 2000, the date of seeding was from November 29, 2000 to December 2 2000, and the date of examination of germination was from March 27 to 29, 2001.

In Table 11, the date of crossing was from May 25, 2001 to June 19, 2001 the date of examination was the last ten days of November 2001, the date of bolling was November 28, 2000, the date of seeding was from December 3 to 8, 2001, and the date of examination of germination was from March 31, 1998 to April 4, 2001.

The crossing of the five combinations shown in Table 10 and Table 11 were conducted utilizing stored pollen from the last ten days of May to the middle ten days of June.

**Table 9**

| Combination of Crossing | No. of Crossing | Bolling No.(%) | Total Seed No. | Seeding No.(%) | Germination Rate(%) |
|---|---|---|---|---|---|
| R.eriocarpum x Kurume azalea "Imashoujou" | 51 | 33 (64.7) | 18150 | 2000 | 50.3 |
| R.eriocarpum x Kurume azalea "Miyagino | 44 | 36 (81.8) | 17640 | 2000 | 53.0 |
| R.eriocarpum x Kurume azalea "Susogo no Ito" | 43 | 21 (48.8) | 6825 | 2000 | 42.5 |
| R.eriocarpum x Kurume azalea "Kirin" | 34 | 24 (70.6) | 22800 | 2000 | 59.8 |
| R.eriocarpum x Kurume azalea "Kure no Yuki" | 50 | 34 (68.0) | 22100 | 2000 | 39.8 |

In any combination of the five combinations of crossing shown in Table 9, the bolling rate was from approximately 50 to 90%, incompatibility of crossing was not observed. As for the germination rate, although the combination of crossing R. eriocarpum x "Kurume Azalea Kure No Yuki" was 39.8, which was relatively low, and the combination of crossing R. eriocarpum x Kurume Azalea "Susogo no Ito" was 42.5, which was also relatively, low, the value of germination rates of other three combinations were from approximately 50% to 60% (Table 9). The combination of R. eriocarpum x Kurume Azalea "Miyagino" having a high bolling rate showed a germination rate of 53%.

**Table 10**

| Combination of Crossing | No. of Crossing | Bolling No.(%) | Total Seed No. | Seeding No.(%) | Germination Rate(%) |
|---|---|---|---|---|---|
| R.eriocarpum x Kurume azalea "Imashoujou" | 20 | 13 (65.0) | 9750 | 2000 | 63.3 |
| R.eriocarpum x Kurume azalea "Miyagino | 20 | 14 (70.0) | 12600 | 2000 | 48.2 |
| R.eriocarpum x Kurume azalea "Susogo no Ito" | 20 | 11 (55.0) | 3850 | 2000 | 60.3 |
| R.eriocarpum x Kurume azalea "Kirin" | 20 | 15 (75.0) | 16500 | 2000 | 72.7 |
| R.eriocarpum x Kurume azalea "Kure no Yuki" | 20 | 14 (70.0) | 10200 | 2000 | 69.6 |

**Table 11**

| Combination of Crossing | No. of Crossing | Bolling No. (%) | Total Seed No. | Seeding No. (%) | Germination Rate(%) |
|---|---|---|---|---|---|
| R.eriocarpum x Kurume azalea "Imashoujou" | 19 | 12 (62.0) | 9200 | 2560 | 66.3 |
| R.eriocarpum x Kurume azalea "Miyagino | 18 | 11 (61.1) | 9350 | 2560 | 60.5 |
| R.eriocarpum x Kurume azalea "Susogo no Ito" | 20 | 11 (55.0) | 4620 | 2560 | 49.6 |
| R.eriocarpum x Kurume azalea "Kirin" | 21 | 13 (61.9) | 12350 | 2560 | 70.1 |
| R.eriocarpum x Kurume azalea "Kure no Yuki" | 20 | 13 (65.0) | 11050 | 2560 | 62.0 |

As can be seen from Table 10 and Table 11, although the combination of R. eriocarpum x Kurume Azalea "Susogo no Ito" showed a bolling rate of 55%, which was relatively low, others showed a bolling rate of from approximately 65% to 75%, in any year and any combination, and thus, imcompatibility of crossing was not observed. As for the germination rate, although the combination of R. eriocarpum x Kurume Azalea "Miyagino" shown in Table 10, and the combination of R. eriocarpum x Kurume Azalea "Susogo no Ito" were as low as 50%, which was relatively low, other combinations of crossing showed a high value of not less than 60% (Table 10, and Table 11). In the case where Kurume azalea was used as a pollen parent, it has been discovered that since they are the same at the variety level, both of the bolling rate and the germination rate show resembling levels among the combinations of crossing.

### [Example 14]

From the last ten days of March to the middle ten days of April of 1997, 2000, and 2001, the crossing of the following five combination was conducted.
(1) R. eriocarpum Nakanoshima Kagoshina, potted) x Kurume Azalea Imashoujou" (cinnabar red, double flower, open field)
(2) R. eriocarpum (Nakanoshima Kagoshina, potted) x Kurume Azalea "Miyagino" (purple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Homare No Yuki" (white, double flower, potted).

As for crossed seed at 1997, the change in survival rate after first and second plantation conducted from the last ten days of March to the first ten days of April 1998 and 1999, respectively was examined. The results are shown in Table 12 and Table 13, respectively. In the first plantation, 120 individuals were planted per one plant bed. In the second plantation after one year, each one individual was planted on a vinyl-made pot. The survival rate in Table 12 showing the first plantation described within parentheses was expressed by taking the number of plantation planted on April 1, 1998 as 100%. The date of plantation was from March 24, 1998 to April 1, 1998 (First Plantation), and the date of examining survival rate was approximately the last date of the examination month as shown in Table 12. The survival rate in Table 13 described within parentheses was expressed by taking the number of plantation planted on April 1, 2002 as 100%. The date of plantation was from March 26 to 29, 1999 (Second Plantation), and the date of examining survival rate was approximately the last date of the examination month as shown in Table 13.

**Table 12**

| Line | Planting No. (Apr.) | Survival No. (rate) | | | | | |
|---|---|---|---|---|---|---|---|
| | | May | Jun. | Sept. | Nov. | Jan. | Mar. |
| R.eriocarpumx Kurume azalea "Imashoujou" | 720 | 706 (98) | 619 (86) | 468 (65) | 432 (60) | 432 (60) | 418 (58) |
| R.eriocarpum× Kurume azalea "Miyagino | 720 | 691 (96) | 598 (83) | 475 (66) | 439 (61) | 425 (59) | 410 (57) |
| R.eriocarpumx Kurume azalea "Susogo no Ito" | 720 | 706 (98) | 583 (81) | 425 (59) | 367 (51) | 338 (47) | 310 (43) |
| R.eriocarpumx Kurume azalea "Kirin" | 720 | 634 (88) | 562 (78) | 439 (61) | 418 (58) | 425 (59) | 389 (54) |
| R.eriocarpum× Kurume azalea "Kure no Yuki" | 720 | 648 (90) | 425 (75) | 360 (59) | 360 (50) | 360 (50) | 346 (48) |

**Table 13**

| Line | Planting No. (Apr.) | Survival No. (rate) | | | | | | |
|---|---|---|---|---|---|---|---|---|
| | | Jun. | Sep.t | Dec. | Mar. | Jun. | Sept. | Dec. |
| R.eriocarpumx Kurume azalea "Imashoujou" | 131 | 111 (85) | 88 (67) | 86 (66) | 86 (66) | 83 (63) | 69 (53) | 67 (51) |
| R.eriocarpumx Kurume azalea "Miyagino | 145 | 112 (77) | 88 (61) | 86 (59) | 82 (57) | 74 (51) | 61 (42) | 58 (40) |
| R.eriocarpumx Kurume azalea "Susogo no Ito" | 240 | 199 (83) | 149 (62) | 142 (59) | 134 (56) | 132 (55) | 125 (52) | 125 (52) |
| R.eriocarpum× Kurume azalea "Kirin" | 198 | 150 (76) | 137 (69) | 127 (64) | 119 (60) | 117 (59) | 107 (54) | 105 (53) |
| R.eriocarpumx Kurume azalea "Kure no Yuki" | 200 | 150 (75) | 122 (61) | 116 (58) | 116 (58) | 108 (54) | 100 (50) | 94 (47) |

In the first plantation (Table 12), the five combinations of crossing two months after the plantation had high survival rates, which were not less than 88%, but at the last day of September, which was 6 month after the plantation, the survival rates were drastically decreased to be not more than 66%. In the second plantation (Table 13), the five combinations of crossing three months after the plantation had high survival rates, which were not less than 75%, but at the last day of September, which was 6 month after the plantation, the survival rates were drastically decreased to be not more than 69%. However, after October, the survival rates were only gradually decreased. While treatment of plantation and high temperature at a summer season stressed the growth in the seedlings, it has been found from these results that F₁ seedlings acquire heat resistant inheritance.

### [Example 15]

As for crossed seedlings at 2000 of the following five combinations of crossing, after the first plantation, a high temperature treatment was conducted over a period of three months, and the seedlings were examined for survival rate. The results are shown in Table 14.
(1) R. eriocarpum Nakanoshima Kagoshina, potted) x Kurume Azalea Imashoujou" (cinnabar red, double flower, open field)
(2) R. eriocarpum (Nakanoshima Kagoshina, potted) x Kurume Azalea "Miyagino" (purple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Kure No Yuki" (white, double flower, potted). Subsequently, at the last ten days of March 2002, the second plantation to a vinyl-made pot was conducted and the survival rate was examined. The results are shown in Table 15.

The survival rate in Table 14 described within parentheses was expressed by taking the number of plantation planted on April 1, 2001 as 100%. The date of plantation was from March 27 to 29, 2001 (First Plantation), and then high temperature treatment over a period of three months. The date of examining survival rate was approximately the last date of the examination month as shown in Table 14. The survival rate in Table 15 described within parentheses was expressed by taking the number of plantation planted on April 1, 2002 as 100%. The date of plantation was from March 24 to 27, 2001 (Second Plantation), and the date of examining survival rate was approximately the last date of the examination month as shown in Table 15.

**Table 14**

| Line | Planting No. (Apr.) | Survival No. (rate) | | | | | |
|---|---|---|---|---|---|---|---|
| | | May | Jun. | Sept. | Nov. | Jan. | Mar. |
| R.eriocarpumx Kurume azalea "Imashoujou" | 960 | 835 (87) | 624 (65) | 403 (42) | 374 (39) | 365 (38) | 336 (35) |
| R.eriocarpum× Kurume azalea "Miyagino | 960 | 816 (85) | 586 (61) | 432 (45) | 422 (44) | 422 (44) | 403 (42) |
| R.eriocarpumx Kurume azalea "Susogo no Ito" | 960 | 797 (83) | 605 (63) | 384 (40) | 305 (38) | 346 (36) | 346 (36) |
| R.eriocarpum× Kurume azalea "Kirin" | 960 | 749 (78) | 634 (66) | 403 (42) | 403 (42) | 384 (40) | 384 (40) |
| R.eriocarpum× Kurume azalea "Kure no Yuki" | 960 | 826 (86) | 605 (63) | 384 (40) | 384 (40) | 374 (39) | 374 (39) |

**Table 15**

| Line | Planting No. (Apr.) | Survival No. (rate) | | | |
|---|---|---|---|---|---|
| | | Jun. | Sept | Dec. | Mar. |
| R.eriocarpum× Kurume azalea "Imashoujou" | 200 | 150 (75) | 110 (55) | 96 (48) | 84 (42) |
| R.eriocarpum× Kurume azalea "Miyagino | 200 | 162 (81) | 88 (44) | 80 (40) | 72 (36) |
| R.eriocarpumx Kurume azalea "Susogo no Ito" | 200 | 154 (77) | 102 (51) | 100 (50) | 90 (45) |
| R.eriocarpumx Kurume azalea "Kirin" | 200 | 162 (74) | 82 (41) | 70 (35) | 64 (32) |
| R.eriocarpumx Kurume azalea "Kure no Yuki" | 200 | 444 (81) | 88 (44) | 84 (42) | 80 (40) |

In the first plantation (Table 14), the five combinations of crossing two months after the plantation had high survival rates, which were not less than 78%, but at the last day of September, which was 6 month after the plantation, the survival rates were drastically decreased to be from 40 to 42%. However, no drastic decreasing was not observed thereafter, and at the last day of the next March, the survival rate was from 35 to 42% (Table 14). In the second plantation (Table 13), the five combinations of crossing three months after the plantation had high survival rates, which were not less than 74%, but at the last day of September, which was 6 month after the plantation, the survival rates were drastically decreased to be not more than 55%. However, no drastic decreasing was not observed thereafter, and at the last day of the next March, the survival rate was from 35 to 42% (Table 15). It can be assumed that in addition to the plantation, the high temperature treatment and a high temperature at a summer season act as sieving of heat resistance, and from the results, it has been discovered that F₁ seedlings acquire heat resistant inheritance.

### [Example 16]

As for crossed seedlings at 2001 of the following five combinations of crossing:
(1) R. eriocarpum Nakanoshima Kagoshina, potted) x Kurume Azalea Imashoujou" (cinnabar red, double flower, open field)
(2) R. eriocarpum (Nakanoshima Kagoshina, potted) x Kurume Azalea "Miyagino" (purple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Kure No Yuki" (white, double flower, potted), no plantation was conducted, but seedlings thereof directly grown on the cell tray were adjusted to be one or two individuals per one well of the cell tray, and the high temperature treatment was conducted over a period of three months from the first ten days of April 2002. The survival rates thereof are shown in Table 16.

The survival rate in Table 16 described within parentheses was expressed by taking the number of seedlings adjusted on April 1, 2002 as 100%. The date of plantation was from March 24 to 27, 2002 (second plantation), and thereafter, a high temperature treatment was conducted over a period of three months. The date of examining survival rate was approximately last day of the month of the examination date described in Table 16.

**Table 16**

| Line | Planting No. (Apr.) | Survival No. (rate) | | | | | |
|---|---|---|---|---|---|---|---|
| | | May | Jun. | Sept. | Nov. | Jan. | Mar. |
| R.eriocarpum× Kurume azalea "Imashoujou" | 512 | 502 (98) | 435 (85) | 358 (70) | 348 (68) | 348 (68) | 338 (66) |
| R.eriocarpum× Kurume azalea "Miyagino | 512 | 502 (98) | 451 (88) | 389 (76) | 384 (75) | 379 (74) | 364 (72) |
| R.eriocarpum× Kurume azalea "Susogo no Ito" | 512 | 481 (94) | 435 (85) | 358 (70) | 348 (68) | 338 (66) | 328 (64) |
| R.eriocarpum× Kurume azalea "Kirin" | 512 | 492 (96) | 415 (81) | 364 (71) | 358 (70) | 358 (70) | 353 (69) |
| R.eriocarpum× Kurume azalea "Kure no Yuki" | 512 | 456 (89) | 399 (78) | 333 (65) | 328 (64) | 317 (62) | 317 (62) |

As shown in Table 16, while high survival rates of from 89 to 98% were shown in all of the combination of crossing at the last day of May, they were from 62 to 72% at at the last day of the next March, not so decreasing the survival rates. The decreasing of the survival rate has been considered to be plantation rather than the high temperature. As a result it has been discovered that that F₁ seedlings aquire heat resistant inheritance.

### [Example 17]

Heat resistant inheritance was taken from the following five combination of crossing:
(1) R. eriocarpum (Nakanoshima Kagoshina, potted) x Kurume Azalea Imashoujou" (cinnabar red, double flower, open field)
(2) R. eriocarpum (Nakanoshima Kagoshina, potted) x Kurume Azalea "Miyagino" (purple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Kure No Yuki" (white, double flower, potted), and the double flower property of the living F₁ seedlings was examined. The results are shown in Table 2.

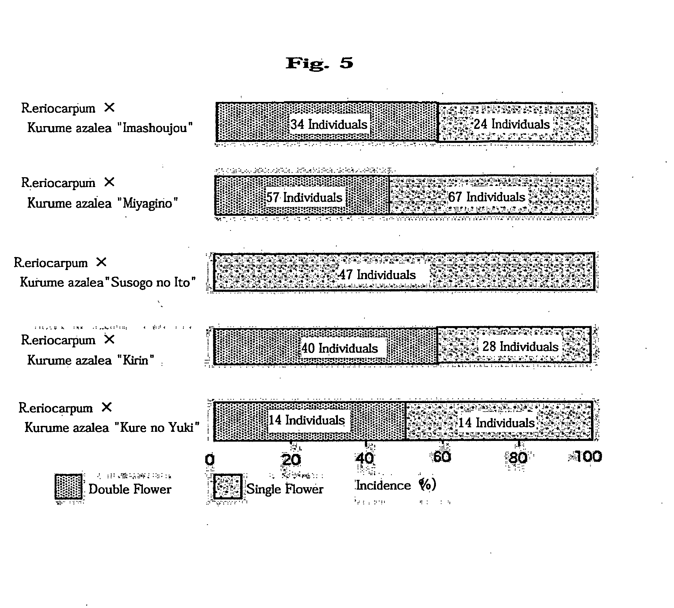

In FIG. 2, 34 double flower F₁ was flowered in individuals of 58 individuals in the combination of R. eriocarpum x Kurume Azalea Imashoujou", 57 individuals of 124 individucals in the combination of R. eriocarpum x Kurume Azalea "Miyagino", 40 individuals of 60 individual in the combination of R. eriocarpum x Kurume Azalea "Kirin", and 14 individuals of 28 individuals in the combination of R. eriocarpum x Kurume Azalea "Kure No Yuki". Proportion of the double flower in the flowering individuals was 58% for the combination of R. eriocarpum x Kurume Azalea "Imashoujou", 46% for the combination of R. eriocarpum x Kurume Azalea "Miyagino", 58% for the combination of R. eriocarpum x Kurume Azalea "Kirin", and 50% for the combination of R. eriocarpum x Kurume Azalea "Kure No Yuki". It is noted that in the combination of R. eriocarpum x Kurume Azalea "Susogo no Ito", all of the 47 flowering individuals were single flower (FIG. 5). In the foregoing combinations of crossing, "Imashoujou", "Miyagino", "Kirin", and "kure no Yuki" which had been used as pollen parents are all double flower, and "Susogo no Ito" is signle flower. Specifically, it has been found, from the proportion of existing double flower in F₁, that double flower property is dominant character, the double flower varieties used as pollen parents have a hetero genotype concerning double flower property. As a result, it has been understood that when a double flower variety is used as a pollen parent, double flower can be obtain at the probability of 50%.

### [Example 18]

Flower color of flower petal of flowering 321 heat resistant azalea was examined for the crossed seeds at 1997. The flower petals were collected and color was measured by a coloriemter to measure L*a*b* value, from which a hue angle (h) was calculated.
(1) R. eriocarpum (Nakanoshima Kagoshina, potted) x Kurume Azalea Imashoujou" (cinnabar red, double flower, open field)
(2) R. eriocarpum (Nakanoshima Kagoshina, potted) x Kurume Azalea "Miyagino" (purple red, double flower, potted)
(3) R. eriocarpum (Nakanoshima Kagoshina, potted) x Kurume Azalea "Susogo no Ito)(Purple, single flower, potted)
(4) R. eriocarpum (Nakanoshima Kagoshina, potted) x Kurume Azalea "Kirin" (Pink, double flower, potted)
(5) R. eriocarpum (Nakanoshima Kagoshina, potted) x Kurume Azalea "Kure No Yuki" (white, double flower, potted).

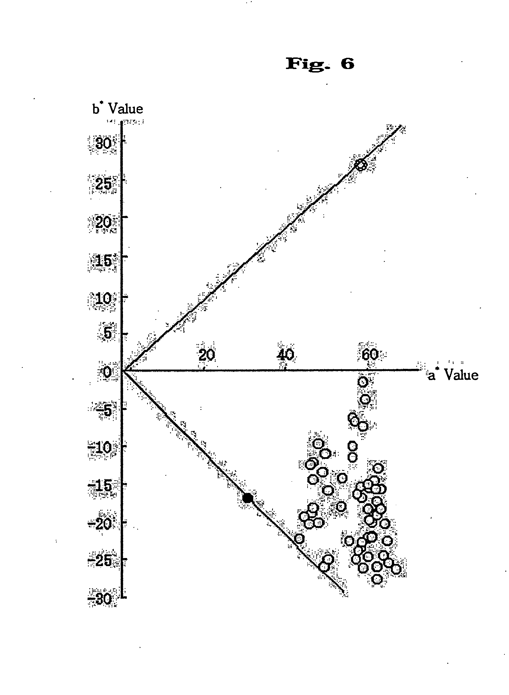

As for crossed seedling of R. eriocarpum x Kurume Azalea "Imashoujou", the flower petal of R. eriocarpum being crossing parent indicated a* value = 28.4 and b* value = -23.2, that of Kurume Azalea "Imashoujou" being a parent indicated a* value = 53.3, and b* value = -23.2. As for the seedlings of F₁ crossing, a* value had individual distribution from 42.4 to 68.0, and b* value was individual distribution from -2.2 to -27.8, and the hue angle h showing the flower color showed a distribution within the scope of the crossing parent. The chromaticity distribution of the crossed seedling was clustered toward R. eriocarpum rather than Kurume Azalea "Imashoujou", indicating that F₁ hybrid has a flower color resembling that of R. eriocarpum (FIG. 6).

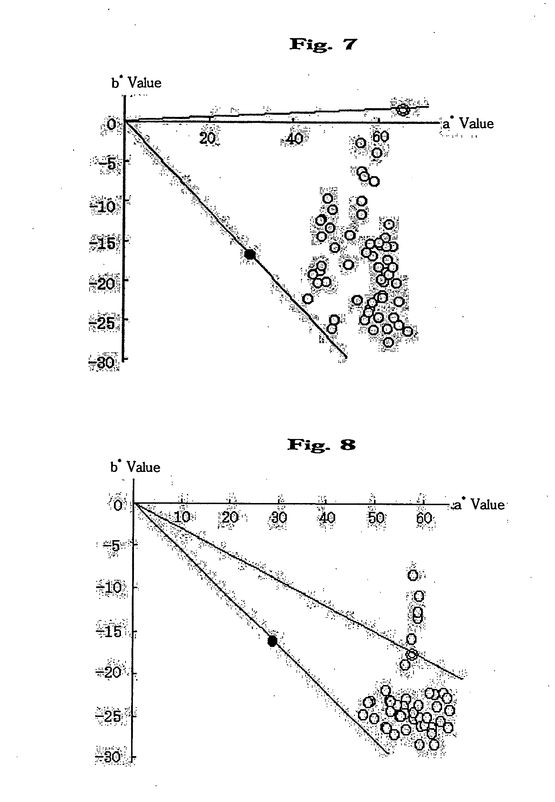

As for corssed seedling of R. eriocarpum x Kurume Azalea "Miyagino", the flower petal of Kurume Azalea "Miyagino" being crossing parent indicated a* value = 66.3 and b* value = -3.0. As for the seedlings of F₁ crossing, a* value had individual distribution from 43.4 to 68.7, and b* value was individual distribution from -2.3 to -26.8, and the hue angle h showing the flower color showed a distribution within the scope of the crossing parent. The chromaticity distribution of the crossed seedling showed a wide distribution from "Miyagino" to R. eriocarpum, but if anything, it was clustered toward R. eriocarpum, indicating that F₁ hybrid has a flower color resembling that of R. eriocarpum (FIG. 7).

As for corssed seedling of R. eriocarpum x Kurume Azalea "Susogo no Ito", the flower petal of Kurume Azalea " Susogo no Ito " being crossing parent indicated a* value = 57.5 and b* value = -17.8. As for the seedlings of F₁ crossing, a* value had individual distribution from 48.0 to 66.3, and b* value was individual distribution from -8.2 to -28.0. As for the the hue angle h, there were those showing the flower color showed a distribution within the scope of the crossing parent, and although it was very minor, those showing a hue angle smaller than that of " Susogo no Ito". The chromaticity distribution of the crossed seedling was clustered toward "Susogo no Ito" rather than R. eriocarpum , indicating that F₁ hybrid has a flower color resembling that of "Susogo no Ito" (FIG. 8) .

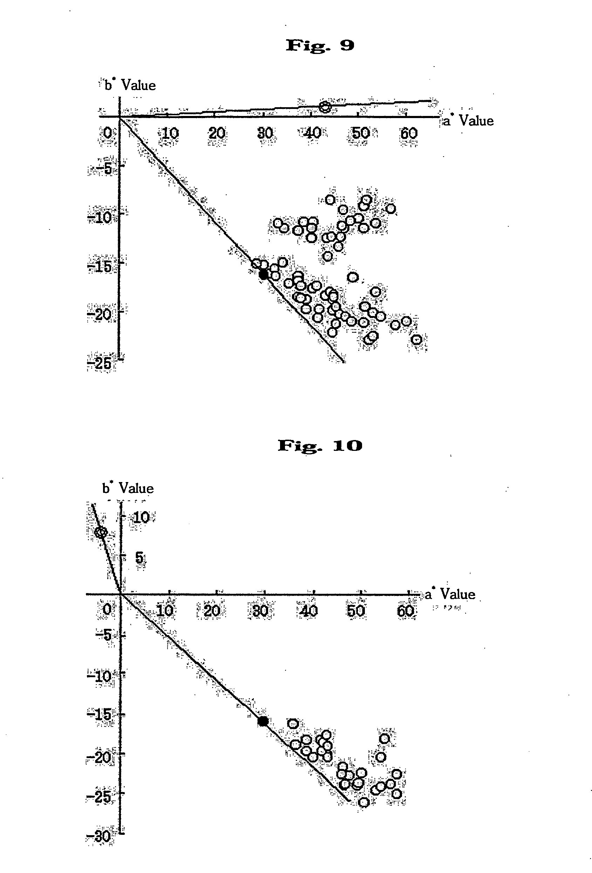

As for crossed seedling of R. eriocarpum x Kurume Azalea "Kirin", the flower petal of "Kirin" being crossing parent indicated a* value = 43.2 and b* value = -2.5. As for the seedlings of F₁ crossing, a* value had individual distribution from 29.1 to 62.0, and b* value was individual distribution from -8.2 to -23.1, and the hue angle h showing the flower color showed a distribution within the scope of the crossing parent. The chromaticity distribution of the crossed seedling was clustered toward R. eriocarpum, indicating that F₁ hybrid has a flower color resembling that of R. eriocarpum (FIG. 9).

As for crossed seedling of R. eriocarpum x Kurume Azalea "Kirin", the flower petal of "Kirin" being crossing parent indicated a* value = 43.2 and b* value = -2.5. As for the seedlings of F₁ crossing, a* value had individual distribution from 29.1 to 62.0, and b* value was individual distribution from -8.2 to -23.1, and the hue angle h showing the flower color showed a distribution within the scope of the crossing parent. The chromaticity distribution of the crossed seedling was clustered toward R. eriocarpum, indicating that F₁ hybrid has a flower color resembling that of R. eriocarpum (FIG. 9).

As for crossed seedling of R. eriocarpum x Kurume Azalea "Kure no Yuki", the flower petal of "Kure no Yuki" being crossing parent indicated a* value = -4.9 and b* value = 7.2. As for the seedlings of F₁ crossing, a* value had individual distribution from 36.3 to 57.7, and b* value was individual distribution from -16.8 to -26.1, and the hue angle h showing the flower color showed a distribution within the scope of the crossing parent. The chromaticity distribution of the crossed seedling was clustered toward R. eriocarpum, indicating that F₁ hybrid has a flower color resembling that of R. eriocarpum (FIG. 10).

### [Example 19]

The crossing of the flowing five combinations was conducted from the last ten days of August 1997 to the middle ten days of September 1997:
(1) R. oldhamii (potted) x Hirado Azalea "Oomurasaki" (Purple red, open field)
(2) R. oldhamii (potted) x Hirado Azalea "Akebono" (Pink, open field)
(3) R. oldhamii (potted) x Hirado Azalea "Shirotae" (White, open field)
(4) R. oldhamii (potted) x Hirado Azalea "Shounoshin" (Cinnabar red, open field)
(5) R. oldhamii (potted) x Kurume Azalea Large Flower "Miyonosakae" (Hybrid of Hirado azalea "Shirotae" xAlalea "Oukan", Pale pink, open field)

Hirado azalea, which flowered at the middle ten days of April, 1997 was used as a pollen parent, and R. oldhamii which flowered at the last ten days of August 1997 was used as a seed parent. Anther from which azalea pollen was collected small flower was packed into paper for wrapping individual doses of powdered medicine, was introduced a glass bin having silica gel contained therein, and was dried for three days. Thereafter, the anther was stored in a refrigerator at a temperature of -20 degree C to be ready for use. The crossing was carried out within a glass chamber which was not warmed. First petal and stamen were removed, and after two or three days, it was confirmed mucilage was sufficiently secreted from the edge of the stamen, and the pollen which had been stored was applied to the stigma. Five to six months after azaleas were crossed, capsules which had been sufficiently matured and when epicarp became brown, were collected, dried at a room temperature to be cleaved. Subsequently, the resulting seeds were packed into paper for wrapping individual doses of powdered medicine, they were introduced into a glass bin having silica gel contained therein, and stored at 4 degree C until seeding.

The crossed seeds were seeded according to the following method from January 18 to 20, 1998. A soil prepared by mixing fine granules of Akadama soil, fine granules of Kanuma soil, and fine granules of Bora soil in a proportion of 1:1:1 was poured into a Jiffy pot (having 8.5 cm in a diameter) to seven-tenth at a first of December, and approximately 0.5 cm of finely divided sphagnum was placed thereon, on which the crossed seeds were seeded.

The soil was previously conducted sterilization and destroying pests with a 1000 times diluted liquid of Benrate (trade name of Dupont) and with a 1000 times diluted liquid of Sumithione (trade name: Kyushu Sankyo Kabushiki Kaisha). Number of seeding was 100 seeds per one pot, and water was well sprinkled after seeding, and pots were transferred within frames covered with transparent vinyl kept at 25 degree C installed within a glass chamber. Germination was observed approximately 20 days after seeding. From immediately after germination to the last ten days of March, 1998 at the time of plantation, a fluorescent lamp for plant was used for lighting 4 hours at midnight (from 10:00 p.m. to 2:00 a.m.) for light supplement. After germination, a liquid fertilizer from fermented oil cake whose N:P:K was formulated to 4:4:3 was given at 1000 time dilution also for water sprinkling per week. Number of crossed flowers, bolling number (rate), total seed number, seeding number, and germination rate were examined. The results are shown in Table 17. The date of crossing was from August 31, 1997 to September 18 1997, the date of examination of bolling was the first ten days of December 1997, the date of bolling was from December 24, 1997 to January 5, 1998, the date of seeding was from January 18 to 20, 1998, and the date of examination of germination was from March 27, 1998 to April 3, 1998.

**Table 17**

| Combination of Crossing | No. of Crossing | Bolling No.(%) | Total Seed No. | Seeding No.(%) | Germination Rate(%) |
|---|---|---|---|---|---|
| R.oldhamii× Hirado Azalea "Oomurasaki" | 26 | 11 (42.3) | 2970 | 2000 | 72.8 |
| R.oldhamii× Hirado Azalea "Akebono" | 25 | 13 (52.0) | 3120 | 2000 | 64.8 |
| R.oldhamii× Hirado Azalea "Shirotae" | 31 | 13 (41.9) | 3250 | 2000 | 65.3 |
| R.oldhamii× Hirado Azalea "Syounoshin" | 29 | 15 (51.7) | 4500 | 2000 | 87.9 |
| R.oldhamii× Kurume Azalea, . Large Flower"Miyonosakae" | 25 | 13 (52.0) | 4680 | 2000 | 82.5 |

In any combination of the five combinations of crossing R. oldhamii x Hirado azale lines, shown in Table 17, the bolling rate was from approximately 42 to 52%, imcompatibility of crossing was not observed. The germination rate was approximately 65% for the combinations of R.oldhamii x Hirado azalea "Akebono", and Hirado azalea "Shirotae", and from 73% to 88% for the crossing combination of R. oldhamii x Hirado azalea "Oomurasaki", R. oldhamii x Hirado azalea "Shounoshin", and R. oldhamii x Kurume Azalea Large Flower "Miyonosakae" (Table 17).

### [Example 20]

The survival rates of progeny F₁ seedlings of the following five combinations crossed from the last ten days of August 1997 to the middle ten days of September 1997 were examined.
(1) R. oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower"Miyonosakae" (Hybrid of Hirado azalea "Shirotae" x Azalea "Oukan", Pale pink, open field).
The seedlings were planted on a plant bed (first plantation) from the last ten days of March 1998 to the first ten days of April 1998, and second plantation was conducted on a 9 cm vinyl-made pot from the last ten days of March 1999 to the first ten days of April 1999. Furthermore, third plantation was conducted on a 15 cm vinyl-made pot from the last ten days of March 2001 to the first ten days of April 2001. The survival rates of F₁ seedlings from April 1998 to March 2003 are shown in Table 18. The survival rate in Table 18 described within parentheses was expressed by taking the number of plantation planted on April 1, 1998 as 100%. The date of plantation was from March 27, 1998 to April 3, 1998 for the first plantation, from March 28, 1999 to April 3, 1999 for second plantation, and from March 30, 2001 to April 2, 2001 for the third plantation. The date of examining the survival rate was approximately the last date of the examination month as shown in Table 18.

**Table 18**

| Line | Planting No. (1998.4) | Survival No. (rate) | | | | |
|---|---|---|---|---|---|---|
| | | 1999. 4 | 2000. 4 | 2001. 4 | 2002. 4 | 2003. 3 |
| R.oldhamii× Hirado Azalea "Oomurasaki" | 120 | 62 (52) | 38 (32) | 32 (27) | 28 (23) | 26 (22) |
| R.oldhamii× Hirado Azalea "Akebono" | 120 | 68 (57) | 40 (33) | 36 (30) | 33 (28) | 32 (27) |
| R.oldhamii× Hirado Azalea "Shirotae" | 120 | 33 (28) | 21 (18) | 19 (16) | 16 (13) | 14 (12) |
| R.oldhamii× Hirado Azalea "Syounoshin" | 120 | 59 (49) | 40 (33) | 37(31) | 33 (28) | 30 (25) |
| R.oldhamii× Kurume Azalea, . Large Flower" Miyonosakae e" | 240 | 129 (54) | 84 (35) | 80 (33) | 75 (31) | 73 (30) |

As for the survival rate at the second plantation one year after the first plantation shown in Table 18, with the exception that the combination of R. oldhamii x Hirado azalea "Shirotae" was 28%, which was very low, other combinations showed from 50% to 57%. At April 2004, the survival rate of the combination R. oldhamii x Hirado azalea "Shirotae"was further decreased to 18%, and other combinations were also similarly decreased to from 32% to 35%. Thereafter, via the third plantation conducted from the last ten days of March 2001 to the first ten days of April 2001 to March 2003, the survival rate was only somewhat decreased. The survival rate of the combination of R. oldhamii x Hirado azalea "Shirotae" was 12% and other combinations was from 22% to 30%.

### [Example 21]

From the last ten days of August 2000 to the middle ten days of September 2000, the crossing of the following five combination was conducted.
(1) R.oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower"Miyonosakae" (Hybrid of Hirado azalea "Shirotae" x Azalea "Oukan", Pale pink, open field).

Hidora azalea, which flowered at from the middle ten days to the last ten days of March 2000, was used as a pollen parent, and R. oldhamii flowering at the last ten days of August was used as a seed parent. Anther from which azalea pollen was collected small flower was packed into paper for wrapping individual doses of powdered medicine, was introduced a glass bin having silica gel contained therein, and was dried for three days. Thereafter, the anther was stored in a refrigerator at a temperature of -20 degree C to be ready for use. The crossing was carried out within a glass chamber which was not warmed. First petal and stamen were removed, and after two or three days, it was confirmed mucilage was sufficiently secreted from the edge of the stamen, and the pollen which had been stored was applied to the stigma. Five to six months after azaleas were crossed, capsules which had been sufficiently matured and which epicarp became brown, were collected, dried at a room temperature to be cleaved. Subsequently, the resulting seeds were packed into paper for wrapping individual doses of powdered medicine, they were introduced into a glass bin having silica gel contained therein, and stored at 4 degree C until seeding.

The crossed seeds were seeded according to the following method from January 21 to 24, 2000. A soil prepared by mixing fine granules of Akadama soil, fine granules of Kanuma soil, and fine granules of Bora soil in a proportion of 1:1:1 was poured into a Jiffy pot (having 8.5 cm in a diameter) to seven-tenth at a first of December, and approximately 0.5 cm of finely divided sphagnum was placed thereon, on which the crossed seeds were seeded.

The soil was previously conducted sterilization and destroying pests with a 1000 times diluted liquid of Benrate (trade name of Dupont) and with a 1000 times diluted liquid of Sumithione (trade name: Kyushu Sankyo Kabushiki Kaisha). Number of seeding was 100 seeds per one pot, and water was well sprinkled after seeding, and pots were transferred within frames covered with transparent vinyl kept at 25 degree C installed within a glass chamber. Germination was observed approximately 20 days after seeding. From immediately after germination to the last ten days of March, 2001 at the time of plantation, a fluorescent lamp for plant was used for lighting 4 hours at midnight (from 10:00 p.m. to 2:00 a.m.) for light supplement. After germination, a liquid fertilizer from fermented oil cake whose N:P:K was formulated to 4:4:3 was given at 1000 time dilution also for water sprinkling per week. Number of crossed flowers, bolling number (rate), total seed number, seeding number, and germination rate were examined. The results are shown in Table 19. In Table 19, the date of crossing was from August 28, 2000 to September 16, 2000, the date of examination of bolling was the first ten days of December 2000, the date of examination of bolling was the first ten days of December 2000, the date of bolling was from December 21 to 24, 2000, the date of seeding was from January 21 to 24, 2001, and the date of examination of germination was from March 26 to 29 2001.

**Table 19**

| Combination of Crossing | No. of Crossing | Bolling No.(%) | Total Seed No. | Seeding No.(%) | Germination Rate(%) |
|---|---|---|---|---|---|
| R.oldhamii× Hirado Azalea "Oomurasaki" | 16 | 10 (62.5) | 7650 | 2760 | 74.7 |
| R.oldhamii× Hirado Azalea "Akebono" | 18 | 11 (61.1) | 9020 | 3150 | 75.7 |
| R.oldhamiix Hirado Azalea "Shirotae" | 14 | 10 (71.4) | 7520 | 2630 | 65.9 |
| R.oldhamiix Hirado Azalea "Syounoshin" | 16 | 12 (75.0) | 4750 | 2030 | 68.7 |
| R.oldhamii× Kurume Azalea, Large Flower" Miyonosakae " | 21 | 13 (61.9) | 7880 | 3340 | 66.6 |

The crossing of the five combinations shown in Table 19 showed the bolling rate of from approximately 60% to 75%, and incompatibility of crossing was not observed. The germination rate was as high as not less than 65% in all of the combinations.

### [Example 22]

The survival rates of progeny F₁ seedlings of the following five combinations crossed from the last ten days of Augut 2000 to the middle ten days of September 2000 were examined.
(1) R.oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower"Miyonosakae" (Hybrid of Hirado azalea "Shirotae" x Azalea "Oukan", Pale pink, open field).
The seedlings were planted on a plant bed (first plantation) from the last ten days of March 2001 to the first ten days of April 2001, and second plantation was conducted on a 9 cm vinyl-made pot from the last ten days of March 2002 to the first ten days of April 2002. The survival rates of F₁ seedlings from April 2001 to March 2003 are shown in Table 20. The survival rate in Table 20 described within parentheses was expressed by taking the number of plantation planted on April 1, 2001 as 100%. The date of plantation was from March 29, 2001 to April 3, 2001 for the first plantation, and from March 25, 2001 to April 1, 2001 for second plantation. The date of examining the survival rate was approximately the last date of the examination month as shown in Table 20.

**Table 20**

| Line | Planting No. (2001. 4) | Survival No. (rate) | |
|---|---|---|---|
| | | 2002 .4 | 2003 .3 |
| R.oldhamiix Hirado Azalea "Oomurasaki" | 480 | 266 (55) | 198 (41) |
| R.oldhamiix Hirado Azalea "Akebono" | 480 | 285 (59) | 225 (47) |
| R.oldhamiix Hirado Azalea "Shirotae" | 480 | 233 (49) | 155 (32) |
| R.oldhamiix Hirado Azalea "Syounoshin" | 480 | 258 (54) | 206 (43) |
| R.oldhamii× Kurume Azalea, Large Flower" Miyonosakae " | 480 | 272 (57) | 255 (53) |

As for the survival rate at the second plantation one yer after the first plantation shown in Table 20, although the combination of R. oldhamii x Hirado azalea "Shirotae" was 49%, which was lower than 50%, other combinations showed from 54% to 59%. At March 2003, the survival rate of the combination R. oldhamii x Hirado azalea "Shirotae" was further decreased to 32%, but in other combinations, there was the combination which maintained the survival rate, such as 53% for R.oldhamii x Kurume Azalea Large Flower "Miyonosakae", and there were combinations which showed somewhat decreased survival rates from 41% to 47%, such as R. oldhamii x Hirado azalea "Akebono", R. oldhamii x Hirado azalea "Oomurasaki", and R. oldhamii x Hirado azalea "Shounoshin".

### [Example 23]

The autumn season flowering properties of progeny F₁ seedlings of the following five combinations crossed from the last ten days of Augut 1997 to the middle ten days of September 1997 were examined.
(1) R.oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower"Miyonosakae" (Hybrid of Hirado azalea "Shirotae" x Azalea "Oukan", Pale pink, open field).
   As a result, F₁ seedlings reached flowering age at August 2001, and started flowering at September. Up to January 2003, individuals which flowerwed from an autumn season to a winter season were 20 indivials (flowering ratio 77%) of 20 individuals for the crossed seedling of R. oldhamii x Hirado azalea "Oomurasaki", 24 indivials (flowering ratio 75%) of 32 individuals for the crossed seedling of R. oldhamii x Hirado azalea "Akebono", 13 individuals (flowering ratio 93%) of 14 individuals for the crossed seedling of R. oldhamii x Hirado azalea "Shirotae", 26 individuals (flowering ratio 87%) of 30 individuals for the crossed seedling of R. oldhamii x Hirado azalea "Shounoshin", and 60 individuals (flowering ratio 82%) of 73 individuals for the crossed seedling of R. oldhamii x Kurume Azalea Large Flower "Miyonosakae". They showed autumn season flowering poperties at a high rate.

### [Example 24]

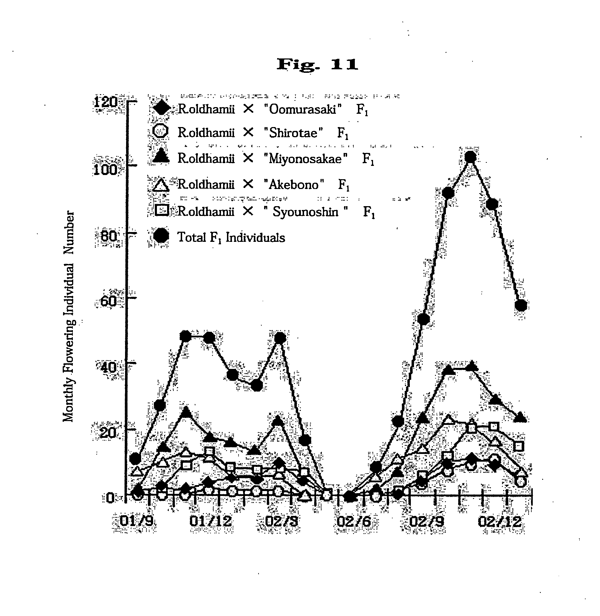

Number of strains (individuals) of progeny F₁ seedlings of the following five combinations crossed from the last ten days of Augut 1997 to the middle ten days of September 1997, which flowered at autumn season was examined (FIG. 11).
(1) R.oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower"Miyonosakae" (Hybrid of Hirado azalea "Shirotae" x Azalea "Oukan", Pale pink, open field)
   Flowering was started at September 2001, the number of the flowering individuals reached maximum at from November to December, and spring season flowering reached a peak at March 2002. On the other hand, at July 2002, flowering was started, and the number of the flowering individuals reached maximum at from October to December. There was a tendency that the peak of autumn (winter) season flowering became faster than the previous year. As a result, F₁ progeny ever-flowering azalea exhibiting summer to winter season flowering property can be obtained from crossing utilizing R. oldhamii as a seed parent at a high rate.

### [Example 25]

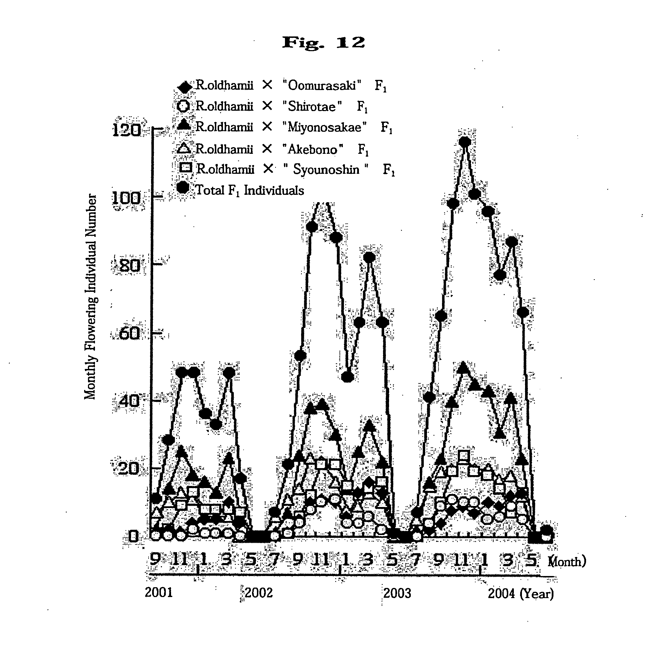

Number of strains (individuals) of progeny F₁ seedlings of the following five combinations crossed from the last ten days of Augut 1997 to the middle ten days of September 1997, which flowered at autumn season was examined (FIG. 12).
(1) R.oldhamii (potted) x Hirado Azalea "Oomurasaki" (Purple red, open field)
(2) R.oldhamii (potted) x Hirado Azalea "Akebono" (Pink, open field)
(3) R. oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large
   Flower"Miyonosakae" (Hybrid of Hirado azalea "Shirotae" x Azalea "Oukan", Pale pink; open field)
   Flowering was started at September 2001, the number of the flowers reached maximum at from November to December, and spring season flowering reached a peak at March 2002. Both at 2002 and 2003, the number of the flower individuals reached maximum, which were 103 individuals and 116 individuals, respectively. At every March from 2002, spring season flowering reached a peak. As a result, it can be understood that F₁ progeny ever-flowering azalea exhibiting summer to winter season flowering property can be obtained from crossing utilizing R. oldhamii as a seed parent at a high rate, and the ever-flowering azalea flowers at four seasons of every year.

### [Example 26]

Flower color of flower petal of flowering 143 ever-flowering azaleas was examined for the crossed from the last ten days of August 1997 to the middle ten days of November 1997, in the following five combinations:
(1) R.oldhamii (potted) x Hirado Azalea "Oomurasaki" (Purple red, open field)
(2) R.oldhamii (potted) x Hirado Azalea "Akebono" (Pink, open field)
(3) R.oldhamii (potted) x Hirado Azalea "Shirotae" (White, open field)
(4) R.oldhamii (potted) x Hirado Azalea "Shounoshin" (Cinnabar red, open field)
(5) R.oldhamii (potted) x Kurume Azalea Large Flower "Miyonosakae" (Hybrid of Hirado azalea "Shirotae" x Azalea "Oukan", Pale pink, open field)
   The flower petals were collected and color was measured by colorimeter. Color is measured with a coloriemter to measure L*a*b* value, from which a hue angle (h) was calculated.

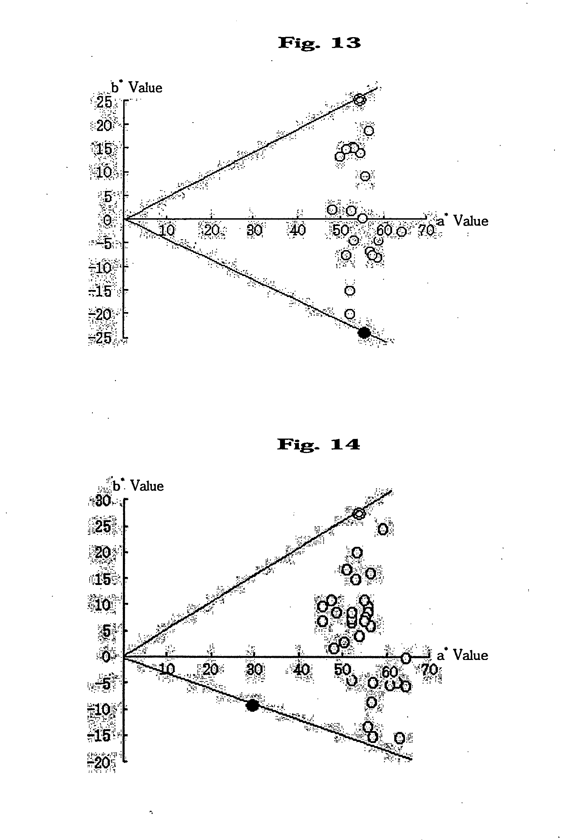

As for crossed seedling of R. oldhamii x Hirado Azalea "Oomurasaki", the flower petal of R. oldhamii being crossing parent indicated a* value = 54.1 and b* value = 25.3, and that of "Oomurasaki" being a parent indicated a* value = 55.8, and b* value =-23.0. As for the seedlings of F₁ crossing, a* value had individual distribution from 48.1 to 65.0, and b* value was individual distribution from 18.2 to -20.1, and the hue angle h showing the flower color showed a distribution within the scope of the crossing parent. The chromaticity distribution of the crossed seedling was widely distributed approximately middle portion between R. oldhamii and "Oomurasaki", and those having flower color resembling R. oldhamii, those having flower color resembling "Oomurasaki", and those having flower color between R. oldhamii and "Oomurasaki" in F₁ hybrids (FIG. 13).

As for crossed seedling of R. oldhamii x Hirado Azalea "Akebono", the flower petal of "Oomurasaki" being a parent indicated a* value = 29.9, and b* value =-8.3. As for the seedlings of F₁ crossing, a* value had individual distribution from 47.7 to 65.0, and b* value was individual distribution from 23.4 to -15.8, and the hue angle h showing the flower color showed a distribution within the scope of the crossing parent. The chromaticity distribution of the crossed seedling was widely distributed approximately middle portion between R. oldhamii and "Akebono", and those having flower color resembling R. oldhamii, those having flower color resembling "Akebono", and those having flower color between R. oldhamii and "Akebono" in F₁ hybrids (FIG. 14).

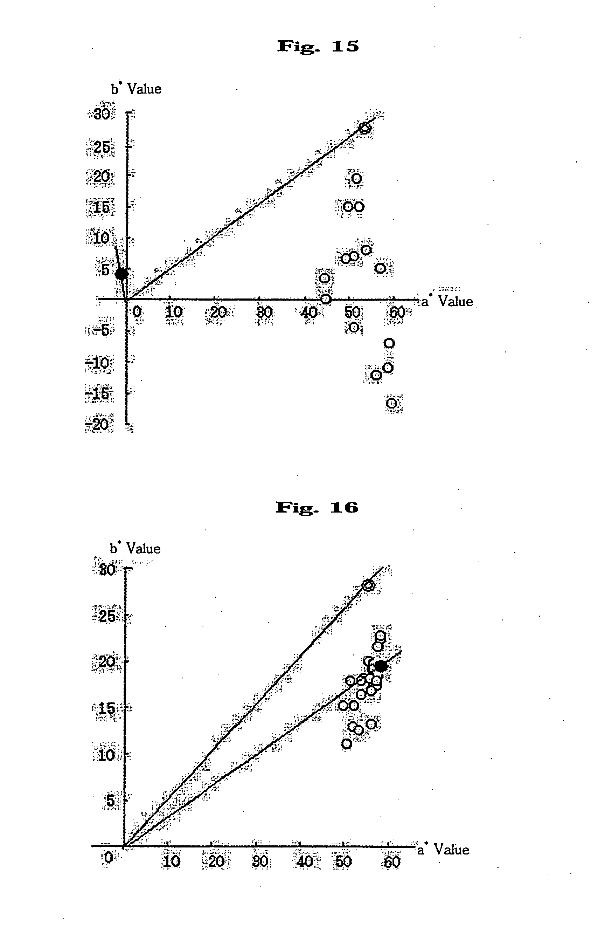

As for crossed seedling of R. oldhamii x Hirado Azalea "Shirotae", the flower petal of "Shirotae" being a parent indicated a* value = 1. 7, and b* value =4 . 9. As for the seedlings of F₁ crossing, a* value had individual distribution from 44.8 to 58.1, and b* value was individual distribution from 19.8 to -16.4, and the hue angle h showing the flower color showed a distribution deviating from the scope of the crossing parent. The chromaticity distribution of the crossed seedling was continuously distributed from cinnabar resembling R. oldhamii to purple red (FIG. 15).

As for crossed seedling of R. oldhamii x Hirado Azalea "Shounoshin", the flower petal of "Shounoshin" being one parent indicated a* value = 58.0, and b* value = 18.4. As for the seedlings of F₁ crossing, a* value had individual distribution from 49.8 to 58.1, and b* value was individual distribution from -10.8 to 22.7. As for the hue angle h showing the flower color, half or more of total were not within hue angle of the crossing parents. The chromaticity distribution of the crossed seedling was clustered toward "Shounoshin" indicating that F₁ hybrid has a flower color resembling that Shounoshin" (FIG. 16).

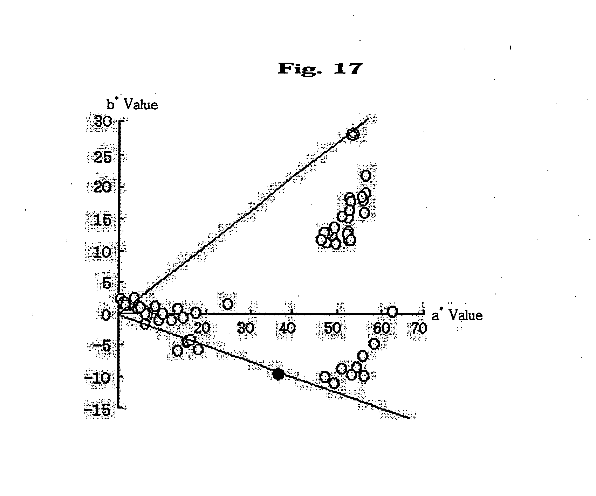

As for crossed seedling of R. oldhamii x Kurume Azalea Large Flower"Miyonosakae", the flower petal of "Miyonosakae" being one parent indicated a* value = 37.2, and b* value = -9.1. As for the seedlings of F₁ crossing, a* value had wide distribution from 0.1 to 61.8, and b* value had wide distribution from 21. 5 to -12.2. These individual distributions were mainly classified into three groups: low a* value/low b* value; high a* value/high b* value, and high a* value/low b* value, which were unique characteristics over other combinations of crossing. The hue angle h showing the flower color showed a distribution deviating from the scope of the crossing parent, and the chromaticity distribution of the crossed seedling had a distribution clustered toward R. oldhamii, a distribution clustered toward "Miyonosakae", and a distribution clustered toward white departing from both parents (FIG. 17).

It is clear from these examples that the method for breeding an azalea according to the present invention is an excellent method for breeding evergreen azalea, heat resistant azalea, and ever-flowering azalea.

### Industrial Applicability

In R. dilatatum which is deciduous, its leaves can enjoy throughout year without falling leaves. In Kurume azalea which cannot withstand poor environments, it does not die within several years even when it is planted on park or pleasure resort at a warm place or a green belt along a road. In the case of potted azalea, even if the flower bud is differentiated and developed by a treatment for enlarged day length and a treatment with growth regulator to overcome dormancy whereby blooming is promoted, it can come into flower at every seasons (ever-flowering).

Based on the discovery that evergreen R. dilatatum, Kurume azalea having applicability to poor environment, and ever-flowering Kurume azalea, which are required from market and which has not yet existed, are bred, the present invention can provide a method for breeding ecotype azalea which introduce into deciduous azalea a gene concerning evergreen, a method for breeding ecotype azalea which introduces into one season flowering azalea a gene concerning ever-flowering, and a method for breeding ecotype azalea which introduces into non-heat resistant azalea a gene concerning heat resistance.

According to the present invention, evergreen R. dilatatum, heat resistant Kurume azalea, and ever-flowering Hirado azalea can be provided.

## Claims

1. A method for breeding an azalea which breeds deciduous azalea with converting it into an evergreen property, in which a gene concerning evergreen is introduced into the deciduous azalea to breed an evergreen azalea.

2. The method for breeding evergreen azalea according to Claim 1, wherein said introduction of the gene is crossing utilizing a evergreen azalea as a flower parent or a seed parent.

3. The method for breeding an azalea according to Claim 1, wherein said deciduous azalea is R. reticulatum, R. dilatatum var. satsumense, or Rhododendron weyrichii, and said evergreen azalea is R. eriocarpum.

4. The method for breeding an azalea according to Claim 1, wherein said crossing is mutual crossing.

5. The method for breeding an azalea according to Claim 1, wherein Rhododendron weyrichii, which is deciduous azalea, and R. dilatatum var. satsumense, which is deciduous azalea are crossed to introduce a gene concerning evergreen is introduced into the deciduous azalea.

6. A method for breeding an azalea which breeds non-heat resistant azalea with converting it into heat resistance, in which a gene concerning heat resistance is introduced into the non-heat resistant azalea to breed a heat resistant azalea.

7. The method for breeding an azalea according to Claim 6, wherein the introduction of said gene is conducted by crossing a heat resistant azalea as a pollen parent or a seed parent.

8. The method for breeding an azalea according to Claim 6, wherein said heat resistant azalea is Rhododendron eriocarpum, and said non-heat resistant azalea is Kurume Azalea or Kurume Azalea Hybrids.

9. The method for breeding an azalea according to Claim 6, wherein said non-heat resistant azalea is evergreen azalea or deciduous azalea, and said heat resistant azalea is deciduous azalea or evergreen azalea, and wherein an evergreen property is imparted to said azalea which is bred with introducing a gene concerning evergreen property into said deciduous azalea.

10. A method for breeding an azalea which breeds one season flowering azalea with converting it into ever flowering property, in which a gene concerning ever-flowering property is introduced into the one season flowering azalea to breed an ever-flowering azalea.

11. The method for breeding an azalea according to Claim 10, wherein a gene which can repeat ever-flowering property every year preferably being introduced into one season flowering azalea.

12. The method for breeding an azalea according to Claim 10, wherein said introduction of the gene is conducted by crossing utilizing ever-flowering azalea as a pollen parent or a seed parent.

13. The method for breeding an azalea according to Claim 10, wherein said ever-flowering azalea is Rhododendron oldhamii, and said one season flowering azalea is Hirado azalea or Hirado Azalea Hybrids.

14. The method for breeding an azalea according to Claim 10, wherein said one season flowering is deciduous azalea or evergreen azalea, and said ever-flowering azalea is evergreen azalea or deciduous azalea, and wherein an evergreen property is imparted to said azalea which is bred with introducing a gene concerning ever-flowering property into said one season flowering azalea.

15. The method for breeding an azalea according to Claim 11, wherein said one season flowering is deciduous azalea or evergreen azalea, and said ever-flowering azalea is evergreen azalea or deciduous azalea, and wherein an evergreen property is imparted to said azalea which is bred with introducing a gene concerning ever-flowering property into said one season flowering azalea.

16. The method for breeding an azalea according to Claim 10, wherein said one season flowering is non heat resistant azalea or heat resistant azalea, and said ever-flowering azalea is heat resistant azalea or non-heat resistant azalea, and wherein an heat resistance is imparted to said azalea which is bred with introducing a gene concerning heat resistance property into said one season flowering azalea.

17. The method for breeding an azalea according to Claim 11, wherein said one season flowering is non heat resistant azalea or heat resistant azalea, and said ever-flowering azalea is heat resistant azalea or non-heat resistant azalea, and wherein a heat resistance is imparted to said azalea which is bred with introducing a gene concerning heat resistance property into said one season flowering azalea.

18. The method for breeding an azalea according to Claim 14, wherein said one season flowering is non heat resistant azalea or heat resistant azalea, and said ever-flowering azalea is heat resistant azalea or non-heat resistant azalea, and wherein a heat resistance is imparted to said azalea which is bred with introducing a gene concerning heat resistance property into said one season flowering azalea.

19. The method for breeding an azalea according to Claim 15, wherein said one season flowering is non heat resistant azalea or heat resistant azalea, and said ever-flowering azalea is heat resistant azalea or non-heat resistant azalea, and wherein a heat resistance is imparted to said azalea which is bred with introducing a gene concerning heat resistance property into said one season flowering azalea.

20. The method for breeding an azalea according to any one of Claims 1, 6, 9-11, and 14-19, wherein the selection of two different azaleas for crossing is conducted by using genotype H^{x}H^{x.} Pg/pg·Cy/cy/Dp/dp which is inheritance of main anthocyanidins, pelargonidin (Pgn), cyanidin (Cyn), and delphinidin (Dpn), concerning the exhibition of flower color.

21. A novel azalea created by the method for breeding an azalea according to any one of Claims 1, 6, 9-11, and 14-19.
